# EUROPEAN PATENT SPECIFICATION

(11) **EP 2 968 426 B1**
(45) Date of publication and mention of the grant of the patent: **07.10.2026**
(21) Application number: 14779967.0
(22) Date of filing: 12.03.2014
(51) Int. Cl.: A61K 36/00, A61K 36/28, A61P 3/10, A61K 38/26, A61K 38/28, A61K 45/06, A61K 31/155, A61K 31/40, A61K 31/4985, A61P 3/04, A61P 43/00, A61P 25/28, A61P 3/00

(54) **PLANT EXTRACTS WITH ANTI-DIABETIC AND OTHER USEFUL ACTIVITIES**
PFLANZENEXTRAKTE MIT ANTIDIABETISCHER WIRKUNG UND ANDEREN NÜTZLICHEN WIRKUNGEN
EXTRAITS DE PLANTE DOTÉE D'ACTIVITÉS ANTI-DIABÉTIQUES ET D'AUTRES ACTIVITÉS UTILES

(30) Priority: 12.03.2013 US 201361777657 P; 12.03.2013 US 201361777927 P
(43) Date of publication of application: 20.01.2016
(60) Divisional application: 26198822.4 / 4 817 680
(73) Proprietor: Housey Pharmaceutical Research Laboratories, L.L.C., Southfield, MI 48076 (US)
(72) Inventor: HOUSEY, Gerard, M., Southfield, MI 48034 (US); BALASH, Monica, Elizabeth, Farmington Hills, MI 48334 (US)
(74) Representative: Witthoff Jaekel Steinecke Patentanwälte PartG mbB
(86) International application number: PCT/US2014/025114
(87) International publication number: WO 2014/165297

(56) References cited:
- WO-A1-2008/121947
- WO-A1-2010/010949
- US-A1- 2003 072 822
- US-A1- 2008 260 924
- US-A1- 2012 100 223
- US-B2- 8 557 512
- DATABASE WPI Week 201212, Derwent World Patents Index; AN 2011-P91172, XP002761318
- DATABASE WPI Week 200416, Derwent World Patents Index; AN 2004-157507, XP002761319
- DATABASE WPI Week 200328, Derwent World Patents Index; AN 2003-279525, XP002761320
- KOUDELA ET AL.: "Nutritional composition and yield of endive cultivars - Cichorium endivia L", HORT. SCI., vol. 35, no. 1, 6 October 2007 (2007-10-06), XP055285770
- KELLY-WELCH: "Transgenic Expression of Insulin Receptor Substrate 2 in Murine B Cells Alters the Cell Density-Dependence of IgE Production In Vitro and Enhances IgE Production In Vivo", J. IMMUNOL., vol. 172, no. 5, 2004, pages 2803 - 2810., XP055285772
- "UNECE STANDARD FFV-22 concerning the marketing and commercial quality control of LETTUCES, CURLED-LEAVED ENDIVES AND BROAD-LEAVED (BATAVIAN) ENDIVES", AGRICULTURAL STANDARDS UNIT TRADE AND TIMBER DIVISION UNITED NATIONS ECONOMIC COMMISSION FOR EUROPE ., 4 January 2011 (2011-01-04), XP055285773, Retrieved from the Internet <URL:http://www.unece.org/fileadmin/DAM/trade/agr/standard/fresh/FFV-Std/English/22Lettuce.pdf>

## Description

This application claims priority to U.S. Application No. 61/777,657, filed March 12, 2013, and U.S. Application No. 61/777,927, filed March 12, 2013.

### FIELD OF THE INVENTION

This invention relates to plant extracts derived from a plant of the plant family Asteraceae containing nutritionally beneficial or medicinally active compounds. Some of these extracts, or the purified compounds contained therein, may be used for the nutritional support, prevention, treatment, or possible cure of various metabolic and other diseases and disorders in human beings and animals, including type 1 and type 2 diabetes, by regulating insulin signaling. This regulatory effect may include modulations of the levels and/or activity of the Insulin Receptor (IR), the Insulin-like Growth Factor (IGF) Receptor, and/or the Insulin Receptor Substrate (IRS) proteins in cells and tissues in the body. A primary focus is directed toward the IRS proteins. Two members of the IRS family of proteins, IRS1 and IRS2, are part of the insulin or insulin-like growth factor signaling pathways, but also mediate signals through other growth factors and cytokines, including IFN-γ, IL-2, IL-4, IL-7, IL-9, IL-13 or IL-15, growth hormone, prolactin, or leptin. IRS1 or IRS2 functional activities also integrate signals emanating from proinflammatory cytokines, including TNF-α, IL-6, IL-1β and related factors. In general proinflammatory cytokines inhibit IRS1/IRS2 signaling which contributes to insulin resistance syndromes.

### BACKGROUND OF THE INVENTION

Diabetes mellitus is a complex and life threatening disease that has been known for more than 2000 years. It occurs in mammals as diverse as monkeys, dogs, rats, mice and human beings. The discovery and purification of insulin in 1921 by Banting and Best and its subsequent therapeutic use in people was a landmark advance in medical science and provided a partial treatment for diabetes that is still in widespread use today. Insulin levels are ordinarily adjusted by the body on a moment to moment basis to keep the blood sugar level within a narrow physiological range. In the diabetic patient, however, periodic insulin injections can only approximate the normal state because the cellular response to insulin in organs and tissues such as liver, muscle and fat is in many cases is also reduced. Consequently, for these and other reasons which will be discussed in detail below, life threatening complications still occur during the lifetime of treated diabetic patients, especially in the case of type 2 (adult-onset) diabetes. (1)

Diabetes arises from various causes, including autoimmune-mediated β-cell destruction (type 1 diabetes); insufficient β-cell insulin secretory capacity to compensate for peripheral insulin resistance (type 2 diabetes); and impaired glucose sensing or insulin secretion (Maturity Onset Diabetes of Youth; MODY)(1). Type 1 Diabetes is genetically complex and caused by circulating auto-antibodies against a variety of islet antigens. Insulin is thought to be one of the principle autoantigens in the pathogenesis of type 1 diabetes, but other antigens deserve attention (2). Since new β-cell formation occurs slowly while type 1 diabetes progresses, it might be necessary to treat the disease by accelerating the rate of β-cell regeneration while attenuating the autoimmune response (3).

Type 2 diabetes is the most prevalent form of diabetes. While it typically manifests at middle age, type 2 diabetes in the developed world is becoming more common in children and adolescents. Physiologic stress-the response to trauma, inflammation, or excess nutrients-promotes type 2 diabetes by activating pathways that impair the postreceptor response to insulin in various tissues (1). Genetic variation also modifies the response to environmental and nutritional factors that promote type 2 diabetes. In a few informative cases, mutations in the insulin receptor or AKT2 explain severe forms of insulin resistance (4). However, common forms of type 2 diabetes are associated with multiple gene variants with modest effects upon insulin action-including peroxisome proliferator-activated receptor gamma (PPARG), peroxisome proliferator activated receptor, gamma, coactivator 1 alpha (PPARGC1A), inward rectifying K+-channel Kir6.2 (KCNJ11), calpain-10 (CAPN10), transcription factor 7-like 2 (TCF7L2), adiponectin (ADIPOQ), adiponectin receptor 2 (ADIPOR2), hepatocyte nuclear factor 4 alpha (HNF4A), uncoupling protein-2, (UCP2), sterol regulatory element binding transcription factor 1 (SREBF1), or high plasma interleukin-6 concentrations (5). Although the effect of each gene is small, these discoveries provide important clues to the pathogenesis of type 2 diabetes.

Regardless of the underlying etiology, dysregulated insulin signaling exacerbated by chronic hyperglycemia and compensatory hyperinsulinemia promotes a cohort of acute and chronic sequela (6). Untreated diabetes progresses to ketoacidosis (most frequent in type 1 diabetes) or hyperglycemic osmotic stress (most frequent in type 2 diabetes), which are immediate causes of morbidity and mortality. In the long term, diabetes is associated with numerous chronic life threatening complications. Due to the marked increase in cerebrovascular disease that occurs in diabetics, the incidence of stroke is as much as three-fold higher than in the non-diabetic population. Similarly, cardiovascular diseases such as peripheral vascular disease, congestive heart failure, coronary artery disease and myocardial infarction are uniformly increased in diabetics as a result of the synergistic effects of hyperglycemia with other cardiovascular risk factors. Furthermore, the combined effects of reduced cardiovascular function and systemic oxidative stress result in damage to capillary endothelial cells in the retina (leading to blindness), mesangial cells of the renal glomerulus causing renal failure, and peripheral nerves resulting in neuropathies causing pain and numbness in the extremities (7).

Diabetes is also associated with age-related degeneration in the central nervous system. Humans beyond 85-90 years of age display less insulin resistance than expected-and centenarians are surprisingly insulin sensitive (8). Compounds that promote peripheral insulin sensitivity and reduce the concentration of circulating insulin required to maintain normal glucose homeostasis provide an ideal treatment of glucose intolerance and its progression to life-threatening diabetes.

### Insulin, Insulin-like Growth Factors, and Receptors

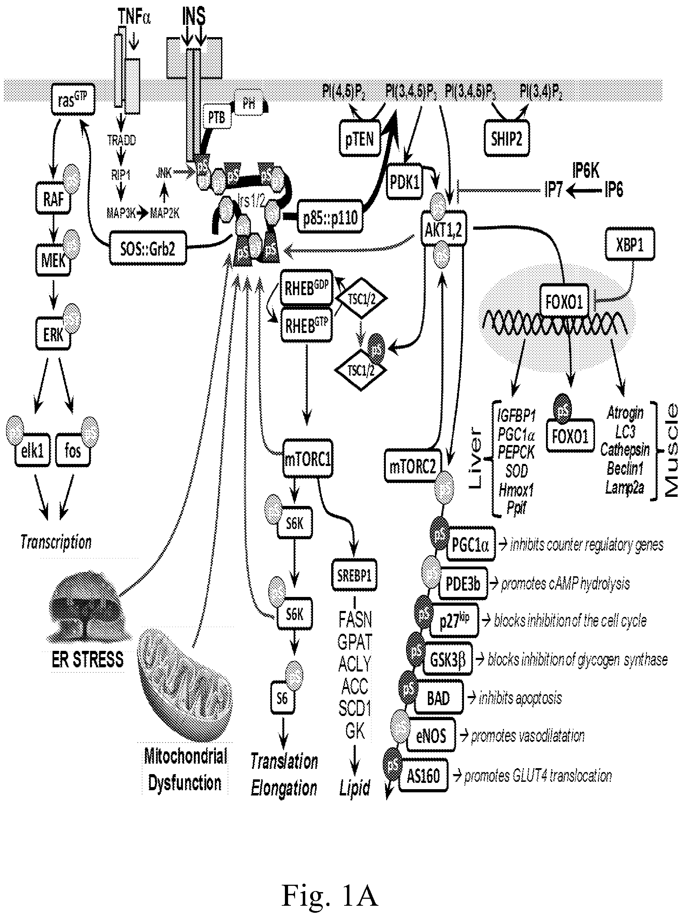

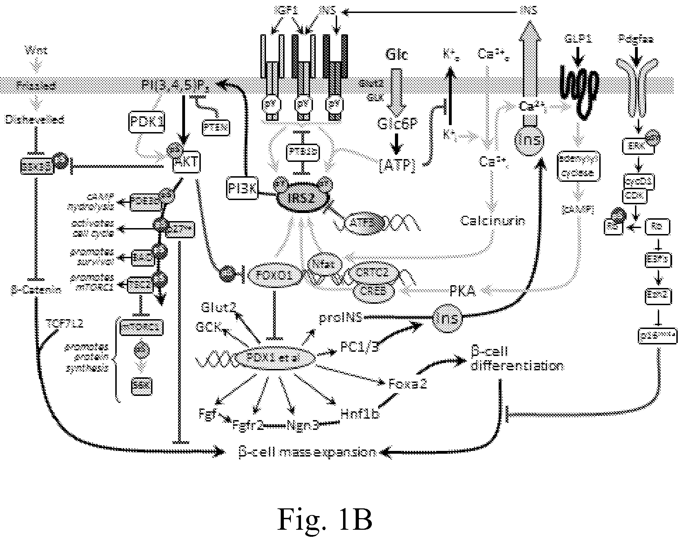

Mammals produce three insulin-like peptides - insulin, insulin-like growth factor-1 (IGF-1) and insulin like growth factor-2 (IGF-2) - which activate five homologous insulin-like receptor tyrosine kinases encoded by the insulin receptor (IR) gene and the insulin-like growth factor-1 receptor (IGF1R) gene (Fig. 1A/B). Insulin is produced in pancreatic β-cells in response to circulating glucose concentrations, whereas endocrine IGF-1 is largely secreted from hepatocytes stimulated by nutrients and growth hormone; IGF-1 and IGF-2 are also produced locally in many tissues and cells, including the central nervous system (9). IGF1 can work coordinately with insulin to regulate nutrient homeostasis, insulin sensitivity and pancreatic β-cell function (9). The insulin receptor and IGF receptor genes encode homologous precursors that form covalently linked dimers that are cleaved by proteolysis to generate a tetramer with two extracellular α-subunits and two transmembrane β-subunits. The extracellular α-subunits create the ligand-binding domain that regulates the activity of the tyrosine kinase on the intracellular portion of the transmembrane β-subunits (10).

High affinity ligand binding induces structural transitions in the catalytic domain of the β-subunit that promote phosphorylation of three tyrosine residues in the kinase regulatory loop (IRa)-Tyr1158, Tyr1162 and Tyr1163 (11). Autophosphorylation releases the regulatory loop from its inhibitory position, which opens the catalytic sites to phosphorylate other proteins (12). The phosphorylated regulatory loop also interacts with other signaling proteins that modulate kinase activity, including Grb10, Grb14, APS and SH2B (13). A fourth tyrosine residue within an NPEY-motif located outside the kinase domain and near the plasma membrane (Tyr960 in IRb; Tyr972 in IRa; Tyr950 in IGF1R) is also phosphorylated, which recruits insulin receptor substrates (IRS-proteins) for tyrosine phosphorylation by the activated receptor kinase (14).

### Insulin Receptor Substrates

Cell-based and mouse-based experiments show that most if not all insulin signals are produced or modulated through tyrosine phosphorylation of IRS1, IRS2 or its homologs; or other scaffold proteins including SHC, CBL, APS and SH2B, GAB1, GAB2, DOCK1, and DOCK2 (15). Although the role of each of these substrates merits attention, work with transgenic mice suggests that many insulin responses-especially those that are associated with somatic growth and nutrient homeostasis-are mediated through IRS1 or IRS2 (1).

The first member of the insulin receptor substrate family of proteins was discovered in 1985, and subsequent research efforts revealed the existence of related IRS family members as well as the signaling pathways to which the IRS proteins are linked. After the discovery that the insulin receptor (IR) possessed a tyrosine kinase enzyme activity, many groups searched for insulin receptor substrates that might regulate downstream signaling from the receptor. The first evidence for the existence of an actual target protein for the insulin receptor, subsequently named an Insulin Receptor Substrate, or "IRS" protein, resulted from the use of phosphotyrosine antibody immunoprecipitates which surprisingly revealed a 185-kDa phosphoprotein (pp185) in insulin-stimulated hepatoma cells (16). Purification and molecular cloning of pp185 revealed one of the first signaling scaffolds as well as the first Insulin Receptor Substrate protein (IRS1) (17,18). IRS1 was determined to be biologically important because it was phosphorylated immediately after insulin stimulation, and catalytically active insulin receptor mutants that failed to phosphorylate IRS1 were biologically inactive.

Several experiments suggested that other related proteins might exist which led to the purification and cloning of Insulin Receptor Substrate 2 (IRS2), a second member of the IRS family (19,20).

Experiments in transgenic mice revealed involvement of IRS1 and IRS2 in promoting somatic growth and nutrient homeostasis. Without IRS1, mice are 50% smaller than normal from birth until they die at 2 years of age. Mice without IRS1 have less body fat and are glucose intolerant. In mice, IRS2 is important for peripheral insulin action, as mice lacking IRS2 display glucose intolerance and hyperlipidemia.

Disruption of the IRS2 gene in mice using standard gene knockout approaches results in diabetes that develops between 8-12 weeks of age. Pancreatic β-cells are lost from these mice as they age, and genes that are important for β-cell function are dysregulated in mice lacking IRS2.

The IRS-proteins are adapter molecules that link the insulin-like receptors to common downstream signaling cascades (Fig. 1A/B). Four IRS-protein genes have been identified in rodents, but only three of these genes (IRS 1, IRS2 and IRS4) are expressed in humans. IRS1 and IRS2 are broadly expressed in mammalian tissues, whereas IRS4 is largely restricted to the hypothalamus and at low levels in a few other tissues. Each of these proteins is targeted to the activated insulin-like receptors through an NH2-terminal pleckstrin homology (PH) domain. The PTB domain binds specifically to the phosphorylated NPEY-motif in the activated receptor kinases (1). The PH domain also promotes the interaction between IRS proteins and the IR, but the mechanism is poorly understood. The PH domain in the IRS-protein plays a specific role as it can be interchanged among the IRS-proteins without noticeable loss of bioactivity, but heterologous PH domains inhibit IRS1 function when substituted for the normal PH domain (21). In addition to the PH and PTB domains, IRS2 also utilizes another mechanism to interact with the activated insulin receptor (22).

### IRS→PI3K→AKT cascade

One of the best studied insulin-like signaling cascades involves the production of PI-3,4,5-P3 by the phosphatidylinositol 3-kinase (PI 3-kinase). The type 1 PI 3-kinase is composed of a regulatory subunit that contains 2 src-homology-2 (SH2) domains and a catalytic subunit that is inhibited by the regulatory subunit until its SH2 domains are occupied by phosphorylated tyrosine residues in the IRS-proteins (23). PI-3,4,5-P3 recruits the Ser/Thr-kinases PDK1 and AKT (also known as PKB) to the plasma membrane where AKT is activated by PDK1-mediated phosphorylation (Fig. 1A/B). AKT phosphorylates many proteins that play a central role in cell survival, growth, proliferation, angiogenesis, metabolism, and migration (24). Phosphorylation of several genuine AKT substrates is especially relevant to insulin-like signaling: GSK3α/β (blocks inhibition of glycogen synthase), AS160 (promotes GLUT4 translocation), the BAD•BCL2 heterodimer (inhibits apoptosis), the FOXO transcription factors (regulates gene expression), p21CIP1 and p27KIP1 (blocks cell cycle inhibition), eNOS (stimulates NO synthesis and vasodilatation), and PDE3b (hydrolyzes cAMP) (Fig. 1A/B). AKT also phosphorylates tuberin (TSC2), which inhibits its GAP activity toward the small G-protein RHEB promoting the accumulation of the RHEB•GTP complex that activates mTOR (24): This pathway provides a direct link between insulin signaling and protein synthesis that is needed for cell growth (Fig. 1A/B).

The role of IRS-proteins in the PI3K→AKT signaling cascade is validated by a wide array of cell-based and mouse-based experiments. Although IRS1 was originally purified and cloned from rat hepatocytes, the principle role of IRS1 and IRS2 during insulin signaling in hepatocytes in vivo was verified only recently (25). The simplest experiments employ an intraperitoneal injection of insulin into ordinary mice, or mice lacking hepatic IRS1 and IRS2. In ordinary mice, insulin rapidly stimulates Akt phosphorylation, and the phosphorylation of its downstream substrates Foxo1 and Gsk3α/β. Both IRS1 and IRS2 must be deleted to uncouple the insulin receptor from the PI3K→AKT cascade (25). These results confirm the shared but absolute requirement for IRS1 or IRS2 for the hepatic insulin signaling.

### Transcriptional regulation of IRS2

The regulation of IRS-protein signaling is an important way to coordinate the intensity and duration of the insulin response among various tissues, but failure of these mechanisms can cause insulin resistance. Transcription of the IRS1 gene is generally stable. By contrast, the production of IRS2 is regulated by multiple nutrient-sensitive transcription factors, including cAMP response element binding protein (CREB) and its binding partner CRTC2, forkhead box O1 (FOXO1), transcription factor E3 (TFE3), and sterol regulatory element binding/factor-1c (SREBF-1c) (26,27). Interestingly, the CREB/CRTC2 transcriptional complex-which binds to cAMP response elements (CRE)-has opposite effects upon IRS2 expression in β-cells and liver. After a meal, the production of ATP from glucose oxidation depolarizes β-cells, which promotes both Ca2+ influx and cAMP production that has many important effects, including the activation of CREB/CRTC2 (26). Thus glucose is coupled directly to IRS2 expression in β-cells, which stimulates β-cell growth and compensatory insulin secretion. By contrast, CREB/CRTC2 promotes IRS2 expression in the fasting liver, which can inhibit the gluconeogenic program by augmenting the basal insulin response.

In addition to cAMP response elements the promoter region of the IRS2 gene includes elements that bind FOXO family members, an E-box that binds TFE3, and a sterol response element (SRE) recognized by SREBF-1c (27). FOXO1 links the PI3K-AKT cascade to the expression of genes important in cell growth, survival, and metabolism. In liver, IRS1 and IRS2 promote the phosphorylation, nuclear export and degradation of FOXO1, which reduces IRS2 expression. Moreover, SREBF-1c concentrations increase during nutrient excess and chronic insulin stimulation, which inhibits FOXO1-mediated IRS2 expression (28). An imbalance in this reciprocal regulation appears to contribute to pathophysiological effects of over-nutrition leading to the development of the metabolic syndrome and diabetes. Thus, compounds that promote IRS2 signaling are expected to have a strong normalizing effect upon hepatic insulin action, especially during nutrient excess.

### Insulin resistance and the dysregulation of IRS-protein signaling.

Insulin resistance is a common pathological state that is associated with many health disorders-obesity, hypertension, chronic infection, dysregulated female reproduction, and kidney and cardiovascular diseases (1). Over the past 15 years, mouse-based experiments have revealed how mutations in genes that mediate the insulin signal, modulate the insulin signal, or respond to the insulin signal contribute to insulin resistance and diabetes. Whereas genetic mutations are obvious sources of life-long insulin resistance, they are usually associated with rare metabolic disorders. Environmental, physiological, and immunological stress causes insulin resistance through heterologous signaling cascades coordinated by complex genetic backgrounds (1).

Obesity is essentially associated with peripheral insulin resistance. Recent studies reveal a variety of factors secreted from adipose tissue that inhibit insulin signaling-FFAs, tumor necrosis factor-alpha (TNFα), and resistin; or factors that promote insulin signaling-adipocyte complement-related protein of 30 kDa (adiponectin) and leptin. Each one of these factors has specific effects upon gene expression patterns that can alter the response of a cell to insulin. However, the effect of these factors upon the expression or function of the IRS-proteins could contribute to the mechanism for insulin resistance (29). Signaling cascades activated during acute trauma or chronic metabolic or inflammatory stress dysregulate IRS-proteins through various mechanisms, including phosphatase-mediated dephosphorylation, proteasome-mediated degradation, and Ser/Thr-phosphorylation. Dysregulation of IRS-protein function also provides a plausible framework to understand the loss of compensatory β-cell function while peripheral insulin resistance emerges (30).

Experiments with TNFα reveal one of the first mechanisms linking inflammatory cytokines to insulin resistance (31). TNFα activates the NH2-terminal JUN kinase (JNK), which phosphorylates IRS 1 on serine residues that inhibit the activation of the PI 3-kinase/Akt pathway in response to insulin. JNK-mediated phosphorylation of IRS1 may also mediate the effects of cellular stress, including endoplasmic reticulum stress. Insulin itself promotes serine phosphorylation of IRS1 through activation of the PI 3-kinase, revealing feedback regulation that might be mediated by many kinases-AKT, PKCC, IKKβ, JNK, mTOR and S6K1 (29).

**The central role of IRS2 signaling in pancreatic β-cells and insulin resistance.**

Mice lacking the gene for Irs1 or Irs2 are insulin resistant, with impaired peripheral glucose utilization. Both types of knockout mice display metabolic dysregulation, but only the Irs2-/- mice develop diabetes between 8-12 weeks of age owing to a near complete loss of pancreatic β-cells (32). This result positions the insulin-like signaling cascade through IRS2 at the center of β-cell function.

Many factors are required for proper β-cell function, including the homeodomain transcription factor Pdx1. Pdx1 regulates downstream genes needed for β-cell growth and function, and mutations in PDX1 cause autosomal forms of early-onset diabetes in people (MODY). Pdx1 is reduced in Irs2-/- islets and Pdx1 haploinsufficiency further diminishes the function of β-cells lacking Irs2. Glucose and glucagon-like peptide-1 have strong effects upon β-cell growth, which depend upon the Irs2 signaling cascade (Fig. 1B). In β-cells, Irs2 is up regulated by cAMP and Ca2+ agonists-including glucose and glucagon-like peptide-1 (GLP1)-which activate cAMP responsive element binding protein (CREB) and the CREB-regulated transcription co-activator 2 (CRTC22) (34). While many cAMP mediated pathways oppose the action of insulin, the up regulation of IRS2 by glucose and GLP1 reveals an unexpected intersection of these important signals (Fig. 1B). Thus, hyperglycemia resulting from the daily consumption of high caloric food promotes β-cell growth, at least in part by increasing IRS2 expression (34). These results suggest that the Irs2-branch of the insulin-like signaling cascade is the "ordinary gatekeeper" for β-cell plasticity and function. Thus compounds that promote IRS2 signaling might have beneficial effects upon beta cell growth, survival and function.

Peripheral insulin resistance contributes to type 2 diabetes, but β-cell failure is an essential feature of all types of diabetes. β-cells frequently fail to compensate for insulin resistance, at least in part because the IRS2-branch of the insulin and IGF signaling cascade which mediates insulin signaling in target tissues also is essential for β-cell growth, function and survival (32).

Because insulin resistance is a cause of metabolic dysregulation and diabetes, understanding its molecular basis is an important goal. Genetic mutations are obvious sources of life-long insulin resistance, but they are associated with rare metabolic disorders and thus difficult to identify in the general population. Inflammation is associated with insulin resistance and provides a framework to understand how diet, acute or chronic stress, and obesity might cause insulin resistance.

Ubiquitin-mediated degradation of IRS-proteins also promotes insulin resistance (Fig. 1A/B). IL6 secreted from leukocytes and adipocytes increases expression of SOCS1 and SOCS3, known for the ability to suppress cytokine signaling. Another function of SOCS1 and SOCS3 is to recruit an elongin BC-based ubiquitin ligase into the IRS-protein complex to mediate ubiquitinylation. Thus, ubiquitin-mediated degradation of IRS-proteins might be a general mechanism of cytokine-induced insulin resistance that contributes to diabetes or β-cell failure (35).

The activity of protein or lipid phosphatases, including PTP1B, SHIP2 or pTEN modulates insulin sensitivity (FIG. 1). Disruption of each of these genes in mice increases insulin sensitivity, suggesting that each might be a target for inhibitor design. PTP1B resides in the endoplasmic reticulum where it dephosphorylates the insulin receptor during internalization and recycling to the plasma membrane (36). This specialized mechanism appears to limit unwanted side effects associated with inhibition of phosphatases, including unregulated cell growth.

### SUMMARY OF THE INVENTION

The present invention relates to the embodiments characterized in the claims and is based on the following observations.

The insulin receptor substrate (IRS) family of proteins that function immediately downstream of the insulin receptor or insulin like growth factor receptors is of central importance in mediating the effects of insulin on responsive cells. In particular, upregulation of the level or functional activity of IRS2 in humans may result in a therapeutically effective chronic treatment as well as a nutritionally beneficial or supportive effect for patients suffering from diabetes, especially the adult onset (type 2) form of the disease, as well as for other disorders in which IRS protein function is insufficient, abnormal or absent altogether. Further, IRS1 and IRS2 are of central importance in mediating signaling through the insulin like growth factor signaling pathway, and signaling by other growth factors and cytokines as well.

Compounds in botanical extracts of the invention may be identified using 32D cells expressing IRS2. Such cells may be created using standard methodology (20). Applicant's have previously created and described a sophisticated target protein specific cell-based assay system capable of identifying IRS2 branch activators of the insulin mediated signal transduction cascade (38). This system is comprised of both Control and Test cells derived from the 32D myeloid progenitor cell line. For the present invention, Applicants designed a cell-based assay system using an IRS2 overproducing histidinol-resistant Test cell line as well as an appropriate his-resistant Control cell line harboring the expression vector only. Under appropriate cultural conditions, the IRS2 overproducing 32D cells become exquisitely sensitive to activation by insulin. Compounds in botanical extracts of the present invention will have a more pronounced effect on the Test cells than on the Control cells, and this effect is quantitated and used to determine the ability of a sample to mimic the effects of insulin as expressed in percentage terms relative to the maximum effect observed following insulin treatment.

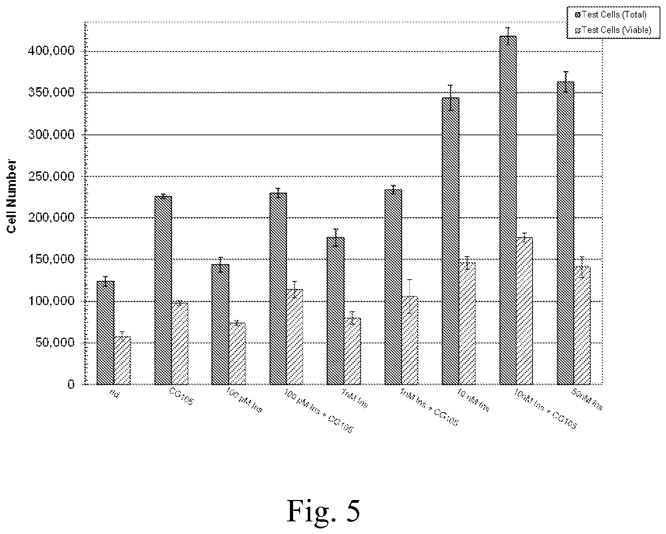

Representative assay results (shown in Tables 1-2 and Figure 5) utilize a 96 well plate format and involve plating Control and Test cells at 25,000 cells per well at time zero. The cells are cultured in IL-3-free medium, and treated for 72 hours with and without 50 nM insulin. Whereas the IRS2-overproducing 32D cell lines become IL-3 independent for the 72 hour duration of the assay (See Figure 5), the Control cells remain absolutely IL-3 dependent and exhibit essentially no cell growth (data not shown). As a result, the assay developed is highly sensitive to compounds (such as insulin) that are capable of activating the IRS2 dependent growth control cascade in IRS2 overproducing 32D cells. Furthermore, potential false positive growth stimulating substances, as emulated by the results of treatment of the cells with IL3, will score positively on the Control cell line as well, and are thus easily eliminated from further consideration and work-up.

This system was utilized to conduct a high throughput screen consisting of more than 100,000 synthetic and natural product derived compounds in search of agents capable of activating the IRS2 signal transduction cascade. A subset of these compounds included extracts derived from a variety of plant species, some of which included edible species. The latter compounds and extracts were screened because Applicants reasoned that compounds derived from edible plants might also contain compounds that are capable of emulating the biological effects of insulin in an IRS2 dependent manner. If such compounds existed within the subset of edible plants contained within the broader plant kingdom, they would provide a basis for understanding at the molecular level why certain diets, such as the Mediterranean diet, have been shown to be associated with a reduced incidence of diabetes, heart disease, and hypertension, leading to corresponding improvements in lifespan and quality of life (40-45, 52).

Some publications have proposed the hypothesis that the benefits of such diets result from a lack of deleterious components present in them, including high fat foods, processed foods, refined sugar, artificial sweeteners and the like. Others have suggested the possibly that protective components such as general antioxidants, or essential nutritional components such as vitamins, or minerals, are the reason why certain diets are beneficial to health and well-being (40-45, 52). Applicants reasoned to the contrary that diets associated with good health and well-being might actually contain pharmacologically active components that synergize with or are otherwise beneficial to, normal cellular functioning in human beings and other mammals. By pharmacologically active, we mean that such active components bind to specific sites on proteins, nucleic acids, or other discrete cellular binding sites and exert pharmacological effects. Remarkably, this theory is demonstrated herein to be true, insofar as these efforts by the Applicants have led to the finding that plants derived from selected species, including : Cichorium endivia, var. latifolium; Lactuca sativa, var. longifolia; Lactuca sativa, var. crispa (See Tables 1 and 2), and others, contain one or more compounds detectable within extracts derived from said species that are capable of substantially activating the IRS2 branch of the insulin mediated signal transduction cascade as determined by the IRS2 target protein specific cell-based assay system described above and previously (38,39). Such compounds may be extracted from the aforementioned plant using an aqueous solvent system, as will be described in detail below. One of skill in the art may utilize alternative extraction methods including, but not limited to, the use of organic or inorganic solvents, and/or supercritical fluid extraction using, for example, carbon dioxide.

### BRIEF DESCRIPTION OF THE FIGURES

Figs. 1A and 1B depict components of the IRS signaling cascade in muscle and liver cells (1A) and pancreatic beta cells (1B), respectively. There are two main limbs that propagate the signal generated through the IRS-proteins: the PI 3-kinase and the Grb2/Sos→ras cascade. Activation of the receptors for insulin and IGF-1 results in tyrosine phosphorylation of the IRS-proteins, which bind PI 3-kinase and Grb2/SOS. The GRB2/SOS complex promotes GDP/GTP exchange on p21ras, which activates the ras→ raf→ MEK-ERK1/2 cascade. The activated ERK stimulates transcriptional activity by direct phosphorylation of elk1 and by phosphorylation of fos through p90rsk. The activation of PI 3-kinase by IRS-protein recruitment produces PI 3,4P2 and PI 3,4,5P3 (antagonized by the action of PTEN or SHIP2), which recruit PDK1 and AKT to the plasma membrane, where AKT is activated by PDK- and mTOR-mediated phosphorylation. The mTOR kinase is activated by RhebGTP, which accumulates upon inhibition of the GAP activity of the TSC1::TSC2 complex by PKB-mediated phosphorylation. The p70s6k is primed through mTOR-mediated phosphorylation for activation by PDK1. AKT phosphorylates many cellular proteins to inactivating PGC1α, p21^{kip}, GSK3β, BAD and AS160, or activate PDE3β and eNOS. The AKT-mediated phosphorylation of the forkhead proteins results in their sequestration in the cytoplasm, which inhibits their influence upon transcriptional activity. Insulin stimulates protein synthesis by altering the intrinsic activity or binding properties of key translation initiation and elongation factors (eIFs and eEFs, respectively) as well as critical ribosomal proteins. This occurs via phosphorylation and/or sequestration of repressive factors into inactive complexes. Components of the translational machinery that are targets of insulin regulation include eIF2B, eIF4E, eEF1, eEF2 and the S6 ribosomal protein (4-6). TNFα activates JNK which can phosphorylate IRS1 inhibiting its interaction with the insulin receptor and subsequent tyrosine phosphorylation. IRS2 expression is promoted by nuclear FOXO, which increases IRS2 expression during fasting conditions. CREB:TORC2 complex also promotes IRS2 expression especially in β-cells, placing IRS2 under the control of glucose and GLP1.

Tables 1 and 2 show the results obtained with a variety of distinct genera and species of edible plants that Applicants' have discovered possess the desired activity. They are ranked according to their ability to stimulate growth of the IRS2-expressing Test cells as normalized to Insulin, with the response elicited by 50 nM Insulin treatment being defined as 100% (Table 1). The activities listed in Table 1 are among the highest that were obtained from numerous experiments. Considerable variations in activity from lot to lot of plant material may be expected depending upon the time of year that the plant was grown and harvested, the degree of freshness of the plant, the soil and climate conditions in which it was propagated, and so on.

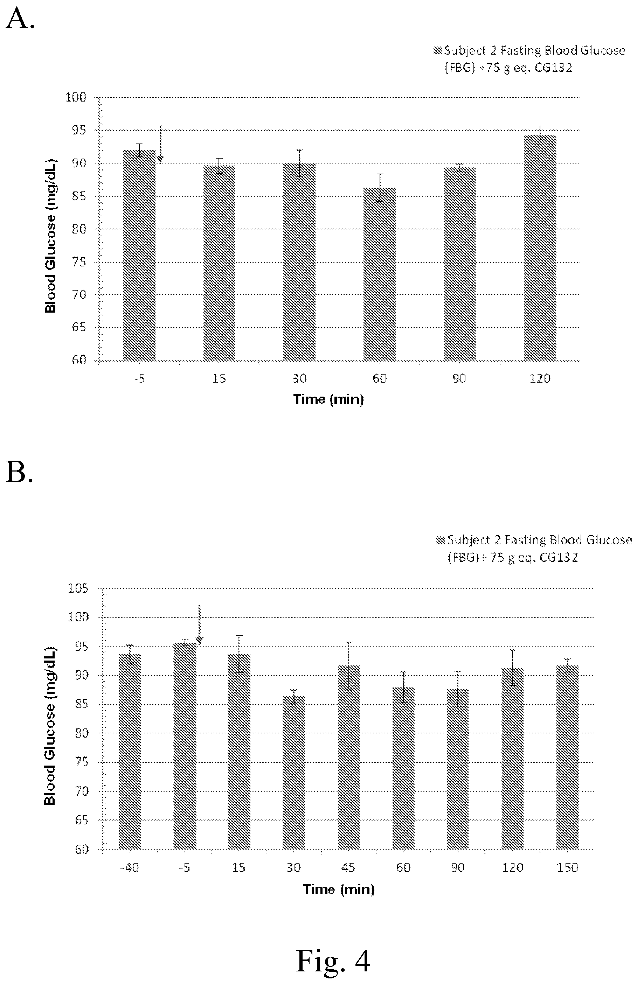

Figures 2, 3 and 4 show the ability of certain extracts to lower fasting blood glucose in normal (non-diabetic) individuals. Either 75 grams of fresh, raw leaf obtained from a local market, or the indicated amount of lyophilized aqueous extract prepared as described herein were consumed as indicated: Figs 2 and 3: CG-105; Fig. 4: CG-132. Blood glucose measurements were determined using a hand-held portable glucose monitor (Abbott Freestyle Freedom Lite). The glucose monitor and the disposable test strips were obtained from a local pharmacy.

Fig. 5. shows selected extracts of the invention enhance the functioning of insulin in the IRS2 overproducing 32D Test cell system such that the amount of insulin necessary to achieve 100% stimulation of growth of the Test cells is lower than it would otherwise need to be in the absence of the selected extract. When CG-105 extract was added to low-dose insulin treatments on the 32D IRS2 Test Cell system, CG-105 extract was found to enhance the activity of insulin at all insulin doses below the maximum insulin stimulating effect (at 50 nM). This activity is variously referred to herein as Insulin Equivalent Activity (IEA) or Insulin Augmenting Activity (IAA), or the additional terms given in paragraphs [51-69] below.

### DETAILED DESCRIPTION OF THE INVENTION

A substantial amount of effort has been made to attempt to identify plant extracts or compounds derived from plants that possess desirable effects for the treatment of human or animal disease. Numerous extracts, drinks, powders, teas, and so forth are marketed with claims relating to providing nutritional support for, or treatment of, many diseases, including diabetes and related metabolic disorders. CN 1 382 734 A discloses the use of bitter chrysanthemum (Cichorium endivia L.) extracts as tea exhibiting hypoglycemic and blood lipid reducing effects. WO 2010/010949 A1 discloses the use of a lipase inhibitor derived from a group of plants including Lactuca sativa and the use thereof in the treatment of postprandial hyperlipemia, obesity and glucose metabolism disorders such as insulin resistance. None of these preparations has been demonstrated to activate the insulin mediated signal transduction cascade in an IRS2-specific manner (46-51, 53,54, 57-70,72, 74,75, 79-81). Zhang et al. conducted a high-throughput screen of more than 50,000 synthetic compounds and natural products and identified a compound that activated the Insulin Receptor (IR). The compound, however, turned out to not be derived from an edible plant source at all. Rather, the compound was derived from a fungal extract (Pseudomassaria) that had been recovered from leaves of an undetermined plant collected near Kinshasa, Democratic Republic of Congo. This work showed, however, that it was at least possible to identify a small molecule capable of having partial activity toward the Insulin Receptor (IR) (71). Prior to their work, it was believed that only a protein hormone such as insulin could activate its cognate receptor.

Pinent et al. demonstrated that a class of compounds known as procyanidins, which are derived from grape seeds, which can induce glucose lowering in an animal model, are able to bind to the IR and at least partially activate the receptor (60, 79). However, the authors concluded that the effects of the procyanidins result in activation of the insulin signaling cascade in a different manner than insulin does. Even with purified fractions of grape seed procyanodin extracts (GSPE), the authors were able to obtain only 40% of the activation of the IR as compared to insulin. In addition, the authors could not establish an IRS2-dependent effect of the compounds (79).

Thus, with the exception of insulin and its corresponding analogs and longacting formulations, no compounds, including proteins, polypeptides or "small molecules" (i.e. molecules having a molecular weight of 2,000 atomic mass units or less) derived from a genus and species known to be edible have been shown to specifically activate the Insulin/Insulin Receptor/IRS2 signal transduction cascade in mammalian cells. In addition, no small molecules have been demonstrated to activate the insulin signaling cascade through an IRS-2 dependent manner. As discussed above in the Background of the Invention, such compounds, extracts, and methods of identifying them from any source would be desirable. This invention provides such extracts derived from selected genera and species of edible plants containing this highly desirable activity.

The invention provides botanical extracts as characterized in the claims useful in a method for the treatment, cure, prevention or nutritional support for various metabolic and other disorders including diabetes, pre-diabetes, metabolic syndrome, obesity, cancer, myelodysplastic syndromes, neurologic disorders such as Alzheimer's disease, dementia and cognitive impairment, attention deficit disorders, premature aging, cardiovascular disorders such as peripheral vascular disease, congestive heart failure, coronary artery disease and myocardial infarction, and others. The disclosure also provides compounds and extracts for the improvement of certain normal resting states such as baseline cognitive status, the cellular aging process, heart rate, stroke volume, blood pressure (systolic and diastolic), blood flow, cardiac output, and the basal metabolic rate of an organism. These beneficial aspects of the invention result, in part, by regulating the level or functional activity of IRS proteins as a result of administering an effective amount of the extracts of the invention to a subject in need or desiring thereof.

In one embodiment, the invention provides botanical extracts for use in restoring or enhancing insulin sensitivity in a cell by upregulating IRS2 function. The invention further provides botanical extracts for use in a method of enhancing pancreatic β-cell function by upregulating IRS2 function. According to the invention, a disease or disorder characterized by reduced or insufficient signaling through IRS2 can be treated by upregulating IRS2 function. Such diseases include, but are not limited to, metabolic disease, diabetes, dyslipidemia, obesity, female infertility, central nervous system disorders, Alzheimer's disease, and disorders of angiogenesis.

According to the invention, upregulation of IRS2 function includes activation of IRS2 or a complex that includes IRS2. In one embodiment of the invention, upregulation of IRS2 function is also accomplished by activation of IRS2 activity, for example by inhibition of phosphorylation of specific serine, threonine or tyrosine residues of IRS2. In another embodiment, upregulation of IRS2 function is accomplished by enhanced expression of IRS2 or by inhibition of degradation of IRS2. In another embodiment, upregulation of IRS2 function is by modulation of a protein or nucleic acid molecule that participates in the mediation of an insulin effect on insulin-responsive cells. Also, modulation of the coupling function of the PH, PTB, or KRLB domains can improve IRS2 function.

More than one hundred samples of various edible plant genera and species were obtained from several local and international markets. Both aqueous and organic extractions of the fruits, leaves, stems and roots of various selected genera of edible plants and other plants were prepared. The extraction procedure was performed as follows: 500 milligrams of fresh plant tissue were pulverized with a mortar and pestle. The ground tissue was then added to 2 mL of water, and homogenized for 1 minute at a setting of 6 using a microprobe (Cole Palmer, LabGen 700). The mixture was then spun at 14,000 RPM for 10 minutes. The supernatant containing the aqueous layer was removed and assayed; whereas the pellet was retained and subjected to organic extraction procedure. Between procedures, samples were maintained at 4 °C to minimize endogenous enzymatic activities.

For larger scale extractions, the procedure was performed as follows: 250 g of wet plant tissue was added to 1L of water, and initial tissue disruption was performed in a table top blender (Kitchen Aid). The blended mixture was then homogenized on ice for 5 minutes at a setting of 20 using a Polytron homogenizer and a standard size probe (Polytron PT2100). The mixture was spun at 10,000 RPM for 10 minutes at 4°C using a JA-10 rotor (Beckman Coulter; Avanti J-25I). The supernatant containing the aqueous layer was removed and assayed. Long term storage was either by refrigeration at 4°C for up to three weeks, or portions of the sample were frozen and lyophilized.

It is preferable to maintain the pH of the extraction solution above 4.3. We have found that pH values of 4.3 and below may cause precipitation of the active factor from crude extracts, leading to a negative result in the 32D IRS2 cell-based assay system. If the solution is brought to pH values higher than 4.3, activity is restored. However, if the active factor is exposed to pH values that are lower (pH approximately 2.0 or less for extended periods), then restoration of activity by subsequently raising the pH is no longer possible, and the active factor becomes essentially irreversibly inhibited.

Under certain conditions, the active principle ("active factor", or simply "factor") obtained from CG-105 is susceptible to heat inactivation, whereas the factor is stable to freezing and lyophilization. Essentially no activity is extractable by the neat organic solvents tested, which included ethanol, methanol, phenol, chloroform, acetonitrile, and benzene.

In addition to emulating the effects of insulin on the Test cell line, it was observed that CG-105 and selected other extracts also increased the overall viability of the cells at the end of the assay on day 3, approximately 72 hours later (Fig. 5).

After the extraction procedure was complete, each extract obtained was assayed either directly using 1 microliter of aqueous extract derived from a 1 liter preparation using 250 grams of fresh plant material, as described above, or after redissolving 5mg of lyophilized powder in 1 ml of distilled water and using 1 microliter per assay well in the 96 well format (approximately 100 microliter total media volume per well). The assay was performed on the 32D Test cell line stably overproducing IRS2 described above and previously (38). The Test cells consisted of 32D cells harboring a histidinol-selectable expression vector and containing a full-length gene encoding murine IRS2 under the transcriptional control of a promoter functional in 32D cells, whereas the Control cells consisted of 32D cells harboring the same histidinol-selectable expression vector lacking the IRS2 coding region.

Tables 1 and 2 shows the activities of several of the most active aqueous extracts obtained from selected species. Activities are reported as a percentage of the total insulin activity obtained using 50 nM Insulin as the positive control for signaling through the IRS2 branch of the signal transduction cascade. Table 2 shows the increase in growth of the Test cells relative to the Control cells for each extract that was tested. (The values indicated are determined by subtracting the mean values of the Control cells from the Test cells for each extract, respectively, as shown in Table 1. (Mean and standard deviations for each extract value are as given in Table 1). As is evident from results shown in Table 2, which are organized taxonomically, certain aqueous extracts exhibit insulin like biological activity in the 32D IRS2 Test cell system equal to as much as 40% of the response obtained with insulin. Positively scoring activities ranged from a low of 10% to a high of 40% of the amount of the cellular response obtained with insulin. One of the families shown in Table 2, the **Asteraceae** family, contains genera and species which uniformly scored positively, though to varying degrees. Other families, such as **Lamiaceae or Brassicaceae,** contained some members which scored positively and others which were negative. Finally, all of the members of the **Amaranthaceae** family that were tested were essentially negative (ND = no activity detected). The botanical extract of the present invention is derived from a plant of the plant family Asteraceae and provides at least 1 x 10⁴ Insulin Equivalent units in 1 milliliter.

| **Table 1 - Natural Product Activity Relative to Insulin** | | | |
|---|---|---|---|
| NP | Control Cells (32D^{his}) | | Test Cells (32D^{IRS2}) |
| **X ≤ 10%** | | | |
| CG148 | -0.1 ± 1.1 | | 3.9 ± 1.3 |
| CG158 | 0.1 ± 2.0 | | 3.9 ± 1.3 |
| CG137 | -0.1 ± 2.9 | | 6.1 ± 1.2 |
| CG126 | 1.6 ± 0.8 | | 6.9 ± 1.4 |
| CG144 | -1.7 ± 0.9 | | 7.3 ± 2.4 |
| CG118 | 0.6 ± 0.1 | | 7.8 ± 0 |
| CG149 | 2.1 ± 1.3 | | 7.8 ± 0 |
| CG128 | 2.0 ± 3.0 | | 8.3 ± 5.6 |
| CG141 | 0.2 ± 0.2 | | 8.5 ± 1.2 |
| CG146 | 1.1 ± 1.3 | | 8.5 ± 1.2 |
| CG154 | 2.5 ± 1.3 | | 9.1 ± 3.9 |
| CG156 | 2.5 ± 0 | | 9.1 ± 1.3 |
| CG142 | 1.0 ± 2.9 | | 9.8 ± 0 |

| **10% < X ≤ 20%** | | | |
|---|---|---|---|
| CG153 | 2.8 ± 2.4 | | 10.4 ± 2.6 |
| CG104 | 3.2 ± 0.4 | | 10.5 ± 2.6 |
| CG113 | 4.3 ± 1.9 | | 10.5 ± 2.6 |
| CG123 | 4.6 ± 1.8 | | 11.8 ± 3.9 |
| CG136 | 0.7 ± 0.4 | | 12.2 ± 0 |
| CG131 | 1.2 ± 2.4 | | 13.9 ± 0 |
| CG159 | 0.8 ± 0.4 | | 14.3 ± 1.3 |
| CG115 | 0 ± 0.7 | | 15.7 ± 0 |
| CG121 | 2.5 ± 1.8 | | 15.7 ± 7.8 |
| CG107 | 4.3 ± 0.8 | | 15.8 ± 7.9 |
| CG112 | 0.4 ± 1.3 | | 15.8 ± 2.6 |
| CG125 | 1.3 ± 1.8 | | 16.7 ± 5.6 |
| CG155 | 1.3 ± 2.5 | | 16.9 ± 1.3 |
| CG114 | 0.4 ± 1.5 | | 17.6 ± 9.8 |
| CG139 | -1.0 ± 1.2 | | 18.3 ± 1.2 |
| CG140 | 0.7 ± 1.1 | | 19.5 ± 2.4 |
| CG157 | 2.0 ± 2.1 | | 19.5 ± 3.9 |

| **20% < X ≤ 25%** | | | |
|---|---|---|---|
| NP | Control Cells (32D^{his}) | | Test Cells (32D^{IRS2}) |
| CG138 | 0.1 ± 1.6 | | 20.7 ± 1.2 |

| **X>25%** | | | |
|---|---|---|---|
| NP | Control Cells (32D^{his}) | | Test Cells (32D^{IRS2}) |
| CG120 | -0.3 ± 0.1 | | 25.5 ± 2.0 |
| CG143 | -2.5 ± 0.3 | | 25.6 ± 3.7 |
| CG132 | -0.6 ± 1.1 | | 26.4 ± 1.4 |
| CG135 | 0 ± 0 | | 26.8 ± 4.9 |
| CG110 | 1.9 ± 2.1 | | 31.6 ± 7.9 |
| CG150 | 2.4 ± 1.0 | | 33.8 ± 5.2 |
| CG105 | 0.6 ± 0 | | 36.8 ± 7.9 |

| **Table 2. Natural Product Activity Relative to Insulin** | | |
|---|---|---|
| **NP** | **Genus_species** | **% Activity above baseline (T_{A}-C_{A})** |
| **Taxonomic Family: Amaranthaceae** | | |
| CG101 | Beta vulgaris | ND |
| CG102 | Beta vulgaris | ND |
| CG103 | Beta vulgaris | ND |
| CG111 | Spinacia oleracea | ND |
| CG116 | Beta vulgaris | ND |
| CG117 | Beta vulgaris | ND |

| **Taxonomic Family: Apiaceae** | | |
|---|---|---|
| CG127 | Petroselium hortense | ND |
| CG133 | Apium graveolens | ND |
| CG134 | Apium graveolens var. dulce | ND |
| CG126 | Petroselinum crispum | 5 |
| CG128 | Coriandrum sativum | 6 |
| CG118 | Petroselinum crispum | 7 |
| CG146 | Anethum graveolens | 7 |
| CG107 | Daucus carota | 12 |
| CG112 | Petroselinum crispum | 15 |

| **Taxonomic Family: Asteraceae** | | |
|---|---|---|
| CG104 | Cichorium endivia | 7 |
| CG123 | Taraxacum officinale | 7 |
| CG131 | Cichorium endivia | 13 |
| CG125 | Lactuca sativa | 15 |
| CG114 | Lactuca sativa | 17 |
| CG120 | Lactuca sativa var. crispa (rd leaf) | 26 |
| CG143 | Cynara scolymus; Cynara cardunculus | 26 |
| CG132 | Lactuca sativa var. crispa (gr. leaf) | 26 |
| CG135 | Artemisia dracunculus | 27 |
| CG110 | Lactuca sativa var. longifolia | 30 |
| CG105 | Cichorium endivia var. latifolium | 36 |

| **Taxonomic Family: Brassicaceae** | | |
|---|---|---|
| CG109 | Brassica oleracea var. italica | ND |
| CG119 | Brassica oleracea var. viridis | ND |
| CG124 | Brassica oleracea var. capitata | ND |
| CG129 | Brassica oleracea var. viridis | ND |
| CG145 | Brassica oleracea var. gemmifera | ND |
| CG156 | Nasturtium officinalis | 7 |
| CG153 | Brassica rapa | 8 |
| CG142 | Eruca sativa | 9 |
| CG136 | Brassica rapa | 12 |
| CG121 | Brassica nigra | 13 |
| CG115 | Brassica rapa chinensis | 16 |

| **Taxonomic Family: Cucurbitaceae** | | |
|---|---|---|
| CG152 | Cucurbita pepo var. pepo | ND |
| CG141 | Cucumis sativus | 8 |

| **Taxonomic Family: Fabaceae** | | |
|---|---|---|
| CG150 | Phaseolus vulgaris | 31 |

| **Taxonomic Family: Lamiaceae** | | |
|---|---|---|
| CG122 | Mentha piperita | ND |
| CG137 | Origanum vulgare | 6 |
| CG154 | Rosmarinus officinalis | 7 |
| CG159 | Ocimum basilicum | 14 |
| CG155 | Salvia officinalis | 16 |
| CG140 | Origanum majorana | 19 |
| CG138 | Thymus vulgaris | 21 |

| **Taxonomic Family: Liliaceae** | | |
|---|---|---|
| CG106 | Allium cepa | ND |
| CG130 | Asparagus officinalis | ND |
| CG113 | Allium porrum | 6 |
| CG139 | Allium schoenoprasum | 18 |

| **Taxonomic Family: Poaceae** | | |
|---|---|---|
| CG158 | Cymbopogon | 4 |
| CG157 | Agropyron Gaertn | 18 |

| **Taxonomic Family: Polygonaceae** | | |
|---|---|---|
| CG148 | Rheum ribes | 4 |

| **Taxonomic Family: Solanaceae** | | |
|---|---|---|
| CG151 | Solanum lycopersicum | ND |
| CG149 | Solanum melongena | 6 |
| CG144 | Capsicum L. | 7 |

| **Taxonomic Family: Vitaceae** | | |
|---|---|---|
| CG147 | Vitis vinifera; Vitis L. | ND |

Based upon the direct comparison to insulin's ability to activate the IRS2 overproducing Test cells, Applicants define the measurement of such activity in the following ways:
Insulin Sensitizing Units - (IS units)
Insulin Sensitizing Activity (ISA units)
Insulin Optimizing Activity - (IOA units)
Insulin Optimizing Units - (IO units)
Insulin Boosting Activity - (IBA units)
Insulin Boosting Units - (IB units)
Insulin Amplifying Units - (IA units)
Insulin Amplifying Activity - (IAA units)
Insulin Intensifying Units - (IIn units)
Insulin Intensifying Activity - (IInA units)
Insulin Augmenting Activity - (IAA units)
Insulin Improving Activity - (IImA units)
Insulin Improving Units - (IIm units)
Insulin Strengthening Units - (ISt units)
Insulin Enriching Units - (IEn units)
Insulin Equivalent Units - (IEq units)
Insulin Equivalent Activity - (IEA units)

**One Unit of Insulin Equivalent Activity** (also known as **Insulin Augmenting Activity)** is defined as the minimum amount of material (compound or extract) necessary to increase the growth of the IRS2 overproducing TEST cells by 1% of the level of growth achieved by treatment of the cells with an appropriate amount of insulin necessary to achieve a substantial increase in growth of the Test cells that a skilled investigator would classify as a sufficient positive control result. This is measured in terms of percentage relative to the maximum effect achieved with Insulin treatment under said positive control conditions. For these purposes, and as shown in the experiments of Tables 1 & 2 and Figure 5, 50 nM insulin is utilized as the positive control. This amount has been determined empirically based upon each lot of insulin that is purchased. By way of example, if 1 microliter of plant extract increases the growth of the IRS2 Overproducing 32D Cell line by 20% in the 96-well plate format assay described above, relative to a positive control comprising 50 nM Insulin treatment (normalized to 100%), then said extract is considered to contain 20 units of Insulin Equivalent (or Insulin Augmenting) Activity. In our experience, 50 to 100 nM Insulin is a saturating amount of insulin under most conditions.

Using standard methodologies for extraction, purification, and filtration, including size-exclusion chromatography, normal and reversed-phase high-pressure liquid chromatography (HPLC), hydrophilic interaction chromatography (HILIC), affinity chromatography, non-sterile and sterile filtration methods, and the like, such activities may be concentrated and increased to as much as 100% of the effect of insulin on the IRS2 Overproducing 32D Cell line (82-84, and references therein). Thus, the disclosure provides a botanical extract that contains at least 1 x 10³ Insulin Equivalent (IE) units per milliliter. In particular, the invention provides a botanical extract derived from a plant of the plant family Asteraceae that contains at least 1 x 10⁴ IE units per milliliter. In one embodiment, the botanical extract contains at least 2 x 10⁴ IE units per milliliter. In still another embodiment, the botanical extract contains at least 3.6 x 10⁴ IE units per milliliter. In yet another embodiment, the botanical extract contains from 1 x 10⁴ to 1 x 10⁵ IE units per milliliter.

This disclosure provides compounds and methods of providing nutritional support, preventing, inducing durable long-term remissions or curing patients with diabetes, metabolic disorders, central nervous system diseases, obesity, fertility and other human disorders as discussed above. The invention is particularly concerned with the IRS proteins and with modulation of the activity of IRS2-mediated cellular signaling pathways as a mechanism for treating human disease and/or providing beneficial nutritional support.

The disclosure also provides an assay to select for specific cultivars with even higher activity or selecting progeny from crosses of cultivars or species for selecting individuals again with high levels of activity. The invention also provides for the use of combinations of two or more individually active species, including variants.

One feature of the present disclosure provides IRS branch activators. The disclosure provides a method of modulating an Insulin Receptor Substrate (IRS) function comprising contacting the IRS (including a cell or tissue comprising the IRS) with a compound, plant fragment, extract of said plant fragment, or an extract derived from the genus and species of plant that has been shown to provide a positive result in the IRS2 specific cell-based assay system described above. Such plants include, but are not limited to, those disclosed in Tables 1 and 2. One possessing a high degree of skill in this field is well aware that genera of plants may evolve independently of one another and yet produce metabolites that are chemically similar or even identical in structure. As an example glucosinolates as a compound class includes more than 100 compounds that are produced by many plants of the Brassicales order which includes more than 4,000 species including mustard, cabbage, broccoli, papaya. A single glucosinolate, such as sulforaphane, is found in high quantities in broccoli but is also present in brussel sprouts, cauliflower, turnip, watercress, bok choy and many other cruciferous vegetables (85). Another example is the production of latex by plants, which occurs in approximately 10% of all plant species, some 40 families including multiple lineages of the two major groups of angiosperms (flowering plants, division Magnoliophyta) the dicots (broad leaf) and monocots (grasses), as well conifers (pine trees, division Pinophyta) and pteridophytes (mosses, ferns, division Pteridophyta). These two examples of compounds being produced by different species, families and genera (for glucosinolates) or further across evolution to different orders, classes and phyla for latex indicate that similar or identical compounds can be produced by unrelated plants across evolution (77,78). Therefore, no limitation is intended on the type of plant extract or purified compound that is capable of providing insulin augmenting activity, wherein such activity may be identified through the use of the IRS2 overproducing Test cells described herein and elsewhere (20, 38). The change in a cellular phenotype (including cell proliferation), activity of the IRS, expression of the IRS, phosphorylation of IRS, or other downstream targets in the IRS2 signal transduction cascade, or binding of the IRS to another insulin receptor or insulin-like growth factor receptor signal transduction pathway component may be monitored. IRS modulation with a compound of the present disclosure may be in treatment or prevention of a4 disease, or in biological assays, cellular assays, biochemical assays, or the like.

Compounds may be identified using 32D cells expressing a selected IRS family member. Such cells may be created using standard methodology originally invented by one of the Applicants and previously described in detail (38). Briefly, 32D cells expressing a selected IRS family member (Test cells) and 32D Cells that essentially do not express the selected IRS family member (Control cells) are brought into contact with a test compound. Compounds of the present disclosure will have a more pronounced effect on the Test cells than on the Control cells.

The present invention also provides extracts for use in methods of preventing, treating, or ameliorating an IRS mediated disease or condition comprising identifying a patient in need, and administering a therapeutically or nutraceutically effective amount of an extract of the present invention. IRS mediated diseases or conditions include, without limitation, diabetes (type 1 and type 2), insulin resistance, metabolic syndrome, dementia, Alzheimer's disease, hyperinsulinemia, dyslipidemia, and hypercholesterolemia, obesity, hypertension, retinal degeneration, retinal detachment, Parkinson's disease, cardiovascular diseases including vascular disease, atherosclerosis, coronary heart disease, cerebrovascular disease, heart failure and peripheral vascular disease in a subject.

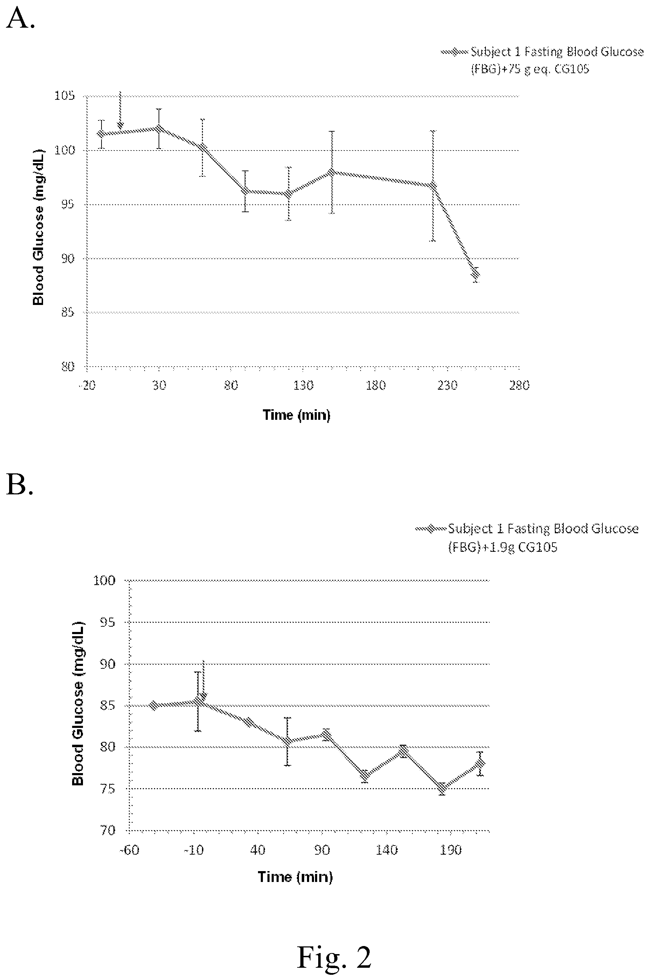

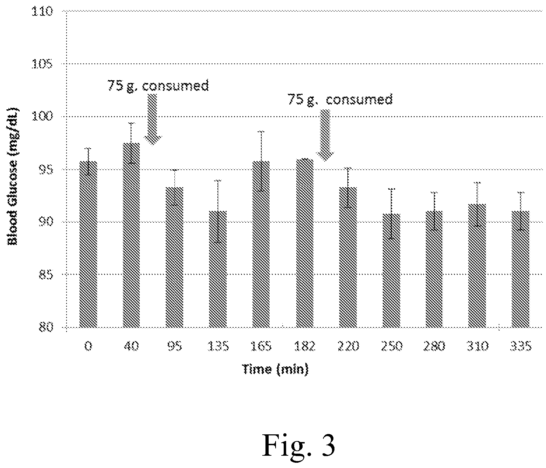

As shown in Figures 2 and 3, a 75g raw dose of a selected genus and species of the plant family Asteraceae (Cichorium endivia, var. latifolium; designated CG-105), when administered orally, lowers fasting blood glucose in a human being. The volunteer test subjects are the Applicants. Arrows indicate time of oral administration. Also shown in Figure 2 are analogous human results of fasting blood glucose before and after administration of a 1.9 gram dose of lyophilized aqueous extract prepared from CG-105 and administered in gelatin capsules. These results demonstrate that this species of plant is capable of inducing a modest fasting blood glucose lowering effect in non-diabetic human beings.

Modulation of IRS function can involve one of the following non-limiting mechanisms. One possible mechanism involves modifying (*i.e.,* promoting or inhibiting) the IRS2 binding interaction with various proteins both upstream and downstream that interact with (bind to) IRS2. These include, for example, the human Insulin Receptor (hIR) which binds to and phosphorylates IRS1 and IRS2, the N-terminal c-jun kinase (JNK), PKC isoforms, ERK1 or ERK2, as well additional upstream or downstream signaling elements such as src homology 2 (SH2) domain-containing proteins that bind to IRS2 and may also phosphorylate, dephosphorylate or otherwise modify IRS.

Another mechanism involves changing the specific pattern of covalent modifications of IRS such as the phosphorylation state of serine, threonine and tyrosine residues, ubiquitination patterns, acetylation or other covalent modifications that alter the function, intracellular localization, or stability of IRS proteins.

A third mechanism involves controlling the expression of the IRS genes in specific cells, including beta cells, brain cells, liver cells muscle cells, reproductive cells and tissues involved in reproduction, fat cells, mammary cells, bone cells and immune system cells, essentially any cells of the body where IRS2 might be naturally expressed. IRS2 is regulated by transcription factors such as CREB, CRTC2, Foxo1, TFE3, and SREBP1. Accordingly, increased IRS2 expression can result from increased activity of the transcription factors that stimulate the transcription of the IRS2 genes. IRS2 expression is also modulated in part by cAMP levels.

IRS is sensitive to proteolytic degradation. Accordingly, compounds that interfere with IRS degradation, for example by interacting with IRS to block degradation or by inhibiting a protease directly, can be used to upregulate IRS signaling activity.

Methods for assessing the effects of compounds on IRS signaling, in vitro and in vivo, are known in the art. For example, cell based assays can be used to confirm increases in IRS signaling. Further, various experimental strategies are available to confirm IRS function, including measuring glucose uptake in response to insulin stimulation, or determining expression of known downstream genes. To observe regulation of IRS expression, reporter genes linked to IRS expression control sequences may be constructed.

Compounds that upregulate the expression or cellular activity of IRS are used to promote IRS signaling. Upregulating IRS in specific tissues can target or prevent diseases involving those specific tissues or cells. For example, upregulation of IRS2 in pancreatic β-cells improves glucose stimulated insulin secretion. Drugs that upregulate the IRS2 gene or promote IRS2 signaling in β-cells will promote β-cell function and are useful to treat or prevent diabetes. Further, the level or functional activity of IRS2 can be modulated in human beings and other mammals in order to ameliorate or prevent the failure or destruction of pancreatic β-cells that causes certain forms of diabetes, and reduce the need for insulin by peripheral insulin sensitive tissues.

IRS genes also functions in peripheral tissues that respond to insulin. Upregulation of the IRS2 gene or upregulation of IRS2 signaling function makes tissues more sensitive to insulin and thus less insulin is needed to elicit an appropriate response. In one embodiment, a single compound promotes IRS2 gene expression or IRS2 function in multiple tissues, for example, promoting insulin secretion in β-cells and insulin sensitivity in other cells and tissues, including, but not limited to, hepatocytes and neurons. In another embodiment, two or more compounds are used to promote IRS activity in different cells or tissues. In some instances, two or more such compounds may be contained within an extract of the present invention derived from a single species of the plant family Asteraceae. These effects of IRS work together to keep glucose under control and prevent diabetes and related disorders that are modulated by IRS function.

Upregulation of IRS expression or an increase of IRS signaling function is also useful to treat other diseases and disorders. Compounds that promote IRS function are useful for reversing catabolism during acute trauma. Insulin resistance is a major problem during acute trauma. Decreased insulin secretion during acute trauma exacerbates autophagy, which increases muscle and tissue wasting that can progress to kidney disease. Insulin resistance and decreased insulin secretion leads to massive catabolism that can threaten survival in the early period of repair. Both processes can be explained in part by the loss of IRS signaling due to inhibition by inflammatory processes and activation of autophagy. Drugs that promote IRS2 function, prevent IRS2 degradation, or promote IRS2 expression reverse these effects.

A major problem with obesity is that peripheral tissues become insulin resistant; if β-cells fail to make enough insulin to overcome the insulin resistance then diabetes develops. Applicants have previously discussed how insulin resistance and diabetes can be treated with compounds that upregulate IRS2 in β-cells and/or peripheral tissues. Upregulating IRS2 in β-cells promotes glucose sensitivity and insulin secretion, and upregulating IRS2 in peripheral tissues reduces the insulin requirements. Accordingly, the incidence of life threatening complications of obesity can be reduced.

Approximately half of the growth of a mouse brain depends on the expression of the IRS2 gene. Drugs that promote IRS2 signaling promote neural growth and regeneration in mammals and people. IRS2 signaling also plays a role in dephosphorylation of the Tau protein, a marker of Alzheimer disease. Upregulation of IRS2 in the hippocampus should promote normal function and contribute to the prevention of the neuronal degeneration associated with Alzheimer disease. Accordingly, extracts of the invention will be beneficial for dementia, including Alzheimer's disease.

IRS2 signaling also plays a role in feeding behavior. Mice lacking IRS2 tend to gain weight as a result of the inability of the brain to properly assess whether insulin has been secreted or not after a meal, so the brain can not determine whether a meal has in fact been consumed. Upregulation of IRS2 in the hypothalamus, and particularly the arcuate nucleus of the hypothalmus, will promote appetite regulation that results in reduced weight gain or even weight loss

IRS2 signaling plays a role in fertility. Notably, female mice lacking IRS2 are infertile. By upregulating IRS2 signaling or IRS2 gene expression in pituitary gonadotrophs or ovaries, ovulation may be enhanced

IRS2 promotes retinal growth. Mice lacking IRS2 display increased loss of retinal neurons, especially rod and cones, leading to blindness. Thus, compounds in botanical extracts of the invention are useful for reducing or preventing retinal degeneration and promoting retinal growth and regeneration.

The invention also provides for coadministration of the extract of the present invention to a subject in combination with a second therapeutic agent or other treatment.

Second therapeutic agents for treatment of diabetes and related conditions include biguanides (including, but not limited to metformin), which reduce hepatic glucose output and increase uptake of glucose by the periphery, insulin secretagogues (including but not limited to sulfonylureas and meglitinides, such as repaglinide) which trigger or enhance insulin release by pancreatic β-cells, and PPARγ, PPARα, and PPARα/γ modulators (e.g., thiazolidinediones such as pioglitazone and rosiglitazone).

Additional second therapeutic agents include GLP1 receptor agonists, including but not limited to GLP1 analogs such as exendin-4 and liraglutide and agents that inhibit degradation of GLP1 by dipeptidyl peptidase-4 (DPP-4). Vildagliptin and sitagliptin are non-limiting examples of DPP-4 inhibitors.

In certain embodiments of the invention, the extracts of the present invention are designed to be coadministered with insulin replacement therapy.

According to the invention, extracts are designed to be coadministered with statins and/or other lipid lowering drugs such as MTP inhibitors and LDLR upregulators, antihypertensive agents such as angiotensin antagonists, e.g., losartan, irbesartan, olmesartan, candesartan, and telmisartan, calcium channel antagonists, e.g. lacidipine, ACE inhibitors, e.g., enalapril, and β-andrenergic blockers (β-blockers), e.g., atenolol, labetalol, and nebivolol.

In another embodiment, a subject is prescribed an extract of the invention in combination with instructions to consume foods with a low glycemic index.

In a combination therapy, the extract is administered before, during, or after another thereapy as well as any combination thereof, i.e., before and during, before and after, during and after, or before, during and after administering the second therapeutic agent. For example, an extract of the invention can be administered daily while extended release metformin is administered daily (55, 56). In another example, an extract of the invention is administered once daily and while exenatide is administered once weekly. Also, therapy with an extract of the invention can be commenced before, during, or after commencing therapy with another agent. For example, therapy with an extract of the invention can be introduced into a patient already receiving therapy with an insulin secretagogue. In addition, extracts of the present invention may be administered once or twice daily in conjuction with other nutritional supplements, vitamins, nutraceuticals, or dietary supplements. Examples include GCE, chlorogenic acid, chicoric acid, cinnamon and various other hydroxycinnamic acids, chromium, chromium picolinate, a multivitamin, and so on.

In another aspect, the present invention provides pharmaceutically acceptable compositions which comprise a therapeutically-effective amount of one or more of the extracts of the present invention, formulated together with one or more pharmaceutically acceptable carriers (additives) and/or diluents. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally.

In another aspect, the present invention provides nutritionally beneficial or supportive compositions which comprise a nutritionally beneficial or supportive amount of one or more of the extracts of the present invention, formulated together with one or more active or inactive ingredients carriers (additives) and/or diluents. As described in detail below, the nutritional supplement formulations of the present invention may be specially formulated for administration in solid or liquid form, including those adapted for the following: (1) oral administration, for example, drinks, foods, chewable pastes or gums, drenches (aqueous or non-aqueous solutions or suspensions), capsules, tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally.

The phrase "therapeutically-effective amount" as used herein means that amount of a composition comprising an extract of the present invention which is effective for producing some desired therapeutic effect in at least a sub-population of cells in an animal at a reasonable benefit/risk ratio applicable to any medical treatment, e.g. reasonable side effects applicable to any medical treatment.

The phrase "nutritionally-effective amount" as used herein means that amount of a composition comprising an extract of the present invention which is effective for producing some desired nutritional effect in at least a sub-population of cells in an animal at a reasonable benefit/risk ratio applicable to any nutritional supplement, e.g. reasonable side effects applicable to any nutritional supplement.

The phrase "composition" whether in singular or plural form, refers both to discrete, chemically defined molecules as well as extracts from plants and other biological organisms containing active ingredients that show a positive result in the IRS2 Cell-based assay system described above.

The phrase "pharmaceutical composition" necessarily includes, when appropriate, nutraceutical compositions, nutritional/dietary supplements, and the like.

The phrase "pharmaceutically acceptable" is employed herein to refer to those compounds, materials, compositions, and/or dosage forms which are, within the scope of sound medical judgment, suitable for use in contact with the tissues of human beings and animals with toxicity, irritation, allergic response, or other problems or complications, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose, cellulose acetate, and hydroxyl propyl methyl cellulose; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

### REFERENCES

1. White MF. Insulin signaling in health and disease. Science 2003 Dec 5; 302 (5651):1710-1.
2. Nakayama M, Abiru N, Moriyama H, Babaya N, Liu E, Miao D, et al. Prime role for an insulin epitope in the development of type 1 diabetes in NOD mice. Nature 2005 May 12; 435 (7039):220-3.
3. Meier JJ, Bhushan A, Butler AE, Rizza RA, Butler PC. Sustained beta cell apoptosis in patients with long-standing type 1 diabetes: indirect evidence for islet regeneration? Diabetologia 2005 Nov; 48 (11):2221-8.
4. Semple RK, Sleigh A, Murgatroyd PR, Adams CA, Bluck L, Jackson S, et al. Postreceptor insulin resistance contributes to human dyslipidemia and hepatic steatosis. J Clin Invest 2009 Feb; 119 (2):315-22.
5. Vaxillaire M, Veslot J, Dina C, Proenca C, Cauchi S, Charpentier G, et al. Impact of common type 2 diabetes risk polymorphisms in the DESIR prospective study. Diabetes 2008 Jan; 57 (1):244-54.
6. DeFronzo RA. Pathogenesis of type 2 diabetes mellitus. Med Clin North Am 2004 Jul; 88 4):787-835, ix.
7. Brownlee M. The pathobiology of diabetic complications: a unifying mechanism. Diabetes 2005 Jun; 54 (6):1615-25.
8. Barbieri M, Rizzo MR, Manzella D, Grella R, Ragno E, Carbonella M, et al. Glucose regulation and oxidative stress in healthy centenarians. Exp Gerontol 2003 Jan; 38 (1-2):137-43.
9. Clemmons DR. Involvement of insulin-like growth factor-I in the control of glucose homeostasis. Curr Opin Pharmacol 2006 Dec; 6 (6):620-5.
10. White MF, Kahn CR. The insulin signaling system. J Biol Chem 1994; 269 (1):1-4.
11. White MF, Shoelson SE, Keutmann H, Kahn CR. A cascade of tyrosine autophosphorylation in the beta-subunit activates the phosphotransferase of the insulin receptor. J Biol Chem 1988 Feb 25; 263 (6):2969-80.
12. Till JH, Ablooglu AJ, Frankel M, Bishop SM, Kohanski RA, Hubbard SR. Crystallographic and solution studies of an activation loop mutant of the insulin receptor tyrosine kinase: insights into kinase mechanism. J Biol Chem 2001 Mar 30; 276 (13):10049-55.
13. Hubbard SR. Crystal structure of the activated insulin receptor tyrosine kinase in complex with peptide substrate and ATP analog. EMBO J 1997; 16 (18):5572-81.
14. White MF, Livingston JN, Backer JM, Lauris V, Dull TJ, Ullrich A, et al. Mutation of the insulin receptor at tyrosine 960 inhibits signal transmission but does not affect its tyrosine kinase activity. Cell 1988; 54:641-9.
15. Yenush L, White MF. The IRS-signaling system during insulin and cytokine action. Bio Essays 1997; 19 (6):491-500.
16. White, M.F., Maron, R. & Kahn, C.R. Insulin rapidly stimulates tyrosine phosphorylation of a Mr-185,000 protein in intact cells. Nature 1985; 318: 183-186.
17. Kahn, C.R., White, M.F., Rothenberg, P.L. Isolated DNA encoding an insulin receptor substrate. US patent No:5,620,200. Filed 10/15/92. Issued 11/9/93.
18. Sun, X.J. et al. Structure of the insulin receptor substrate IRS-1 defines a unique signal transduction protein. Nature 1991; 352: 73-77.
19. White, M.F., Sun, X.J., Pierce, J.H. DNA encoding an insulin receptor substrate. Us patent No:5,858,701. Filed 10/03/94. Issued 01/12/99.
20. Sun, X.J. et al. Role of IRS-2 in insulin and cytokine signalling. Nature 1995; 377: 173-177.
21. Burks DJ, Wang J, Towery H, Ishibashi O, Lowe D, Riedel H, et al. IRS pleckstrin homology domains bind to acidic motifs in proteins. J Biol Chem 1998 Nov 20; 273 (47):31061-7.
22. Wu J, Tseng YD, Xu CF, Neubert TA, White MF, Hubbard SR. Structural and biochemical characterization of the KRLB region in insulin receptor substrate-2. Nat Struct Mol Biol 2008 March 15(3) 251-8.
23. Backer JM, Myers MG, Jr., Shoelson SE, Chin DJ, Sun XJ, Miralpeix M, et al. Phosphatidylinositol 3'-kinase is activated by association with IRS-1 during insulin stimulation. EMBO J 1992; 11:3469-79.
24. Manning BD, Cantley LC. AKT/PKB signaling: navigating downstream. Cell 2007 Jun 29; 129 (7):1261-74.
25. Dong XC, Copps KD, Guo S, Li Y, Kollipara R, DePinho RA, et al. Inactivation of hepatic Foxo1 by insulin signaling is required for adaptive nutrient homeostasis and endocrine growth regulation. Cell Metab 2008 Jul; 8 (1):65-76.
26. Jhala US, Canettieri G, Screaton RA, Kulkarni RN, Krajewski S, Reed J, et al. cAMP promotes pancreatic beta-cell survival via CREB-mediated induction of IRS2. Genes Dev 2003 Jul 1; 17 (13):1575-80.
27. Shimano H. SREBP-1c and TFE3, energy transcription factors that regulate hepatic insulin signaling. J Mol Med 2007 May; 85 (5):437-44.
28. Ide T, Shimano H, Yahagi N, Matsuzaka T, Nakakuki M, Yamamoto T, et al. SREBPs suppress IRS-2-mediated insulin signalling in the liver. Nat Cell Biol 2004 Apr; 6 (4):351-7.
29. Zick Y. Ser/Thr phosphorylation of IRS proteins: a molecular basis for insulin resistance. Sci STKE 2005 Jan 25; 2005 (268):e4.
30. White MF. Regulating insulin signaling and beta-cell function through IRS proteins. Can J Physiol Pharmacol 2006 Jul; 84 (7):725-37.
31. Wellen KE, Hotamisligil GS. Inflammation, stress, and diabetes. J Clin Invest 2005 May; 115 (5):1111-9.
32. Withers DJ, Gutierrez JS, Towery H, Burks DJ, Ren JM, Previs S, et al. Disruption of IRS-2 causes type 2 diabetes in mice. Nature 1998; 391 (6670):900-4.
33. Jonsson J, Carlsson L, Edlund T, Edlund H. Insulin-promoter-factor 1 is required for pancreas development in mice. Nature 1994; 371 (6498):606-9.
34. Weir GC, Bonner-Weir S. A dominant role for glucose in beta cell compensation of insulin resistance. J Clin Invest 2007 Jan;117(1):81-3.
35. Krebs, D.L., Hilton, D.J. A new role for SOCS in insulin action. Suppressor of cytokine signaling. Sci STKE. 2003 Feb 11; (169): PE6.
36. Haj, F.G., Verveer, P.J., Squire, A., Neel, B.G., Bastiaens, P.I. Imaging sites of receptor dephosphorylation by PTP1B on the surface of the endoplasmic reticulum. Science. 2002 Mar 1; 295(5560):1708-1711
37. Berge, S.M., Bighley, L.D., Monkhouse, D.C. Pharmaceutical salts. J Pharm Sci. 1977 Jan; 66(1):1-19.
38. Housey, GM, White, MF, Method of Screening Activators and/or Inhibitors of Insulin Receptor Substrate 2. US Patent 8,557,512 B2. Serial No. 10/541,263. Filed 12/31/2003. Issued 10/15/2013.
39. Housey, G.M., Balash M. (2010) IRS Modulators. US Provisional Patent Application. Filed June 3, 2010.
40. Liu S, Serdula M, Janket SJ, Cook NR, Sesso HD, Willett WC, Manson JE, Buring JE. A prospective study of fruit and vegetable intake and the risk of type 2 diabetes in women. Diabetes Care. 2004 Dec; 27 (12):2993-6.
41. Bazzano LA, Li TY, Joshipura KJ, Hu FB. Intake of fruit, vegetables, and fruit juices and risk of diabetes in women. Diabetes Care. 2008 Jul; 31 (7):1311-7
42. Montonen J, Järvinen R, Heliövaara M, Reunanen A, Aromaa A, Knekt P. Food consumption and the incidence of type II diabetes mellitus. Eur J Clin Nutr. 2005 Mar; 59 (3):441-8.
43. Meyer KA, Kushi LH, Jacobs DR Jr, Slavin J, Sellers TA, Folsom AR. Carbohydrates, dietary fiber, and incident type 2 diabetes in older women. Am J Clin Nutr. 2000 Apr; 71 (4):921-30.
44. Villegas R, Shu XO, Gao YT, Yang G, Elasy T, Li H, Zheng W. Vegetable but not fruit consumption reduces the risk of type 2 diabetes in Chinese women. J Nutr. 2008 Mar; 138 (3):574-80.
45. Carter P, Gray LJ, Troughton J, Khunti K, Davies MJ. Fruit and vegetable intake and incidence of type 2 diabetes mellitus: systematic review and meta-analysis. BMJ. 2010 Aug 18; 341:c4229.
46. Muthusamy VS, Saravanababu C, Ramanathan M, Bharathi Raja R, Sudhagar S, Anand S, Lakshmi BS. Inhibition of protein tyrosine phosphatase 1B and regulation of insulin signalling markers by caffeoyl derivatives of chicory ( Cichorium intybus) salad leaves. Br J Nutr. 2010 Sep; 104 (6):813-23.
47. Ahmed N, Tarannum S. Acetylcholinesterase activity in the brain of alloxan diabetic albino rats: Presence of an inhibitor of this enzyme activity in the cerebral extract. Int J Diabetes Dev Ctries. 2009 Oct; 29 (4):174-7.
48. Muthusamy VS, Anand S, Sangeetha KN, Sujatha S, Arun B, Lakshmi BS. Tannins present in Cichorium intybus enhance glucose uptake and inhibit adipogenesis in 3T3-L1 adipocytes through PTP1B inhibition. Chem Biol Interact. 2008 Jul 10; 174 (1):69-78.
49. Pushparaj PN, Low HK, Manikandan J, Tan BK, Tan CH. Anti-diabetic effects of Cichorium intybus in streptozotocin-induced diabetic rats. J Ethnopharmacol. 2007 May 4; 111 (2):430-4.
50. Petlevski R, Hadzija M, Slijepcević M, Juretić D, Petrik J. Glutathione S-transferases and malondialdehyde in the liver of NOD mice on short-term treatment with plant mixture extract P-9801091. Phytother Res. 2003 Apr; 17 (4):311-4.
51. Petlevski R, Hadzija M, Slijepcevic M, Juretic D. Effect of 'antidiabetis' herbal preparation on serum glucose and fructosamine in NOD mice. J Ethnopharmacol. 2001 May; 75 (2-3):181-4.
52. Estruch R, Ros E, Salas-Salvadó J, Covas MI, D Pharm, Corella D, Arós F, Gómez-Gracia E, Ruiz-Gutiérrez V, Fiol M, Lapetra J, Lamuela-Raventos RM, Serra-Majem L, Pintó X, Basora J, Muñoz MA, Sorli JV, Martinez JA, Martínez-González MA; the PREDIMED Study Investigators. Primary Prevention of Cardiovascular Disease with a Mediterranean Diet. N Engl J Med. 2013 Feb 25.
53. Mascherpa D, Carazzone C, Marrubini G, Gazzani G, Papetti A. Identification of phenolic constituents in Cichorium endivia var. crispum and var. latifolium salads by high-performance liquid chromatography with diode array detection and electrospray ioniziation tandem mass spectrometry. J Agric Food Chem. 2012 Dec 12; 60 (49):12142-50.
54. Papetti A, Daglia M, Gazzani G. Anti- and pro-oxidant water soluble activity of Cichorium genus vegetables and effect of thermal treatment. J Agric Food Chem. 2002 Jul 31; 50 (16):4696-704.
55. Diabetes Prevention Program Research Group. Reduction in the Incidence of Type 2 Diabetes with Lifestyle Intervention or Metformin. N Engl J Med 2002 Feb 7; 346 (6): 393-403
56. Campbell, IW. Metformin - life begins at 50: A symposium held on the occasion of the 43rd Annual Meeting of the European Association for the Study of Diabetes, Amsterdam, The Netherlands, September 2007, The British Journal of Diabetes & Vascular Disease. 2007 Sept 7: 247-252.
57. Papetti A, Daglia M, Aceti C, Sordelli B, Spini V, Carazzone C, Gazzani G. Hydroxycinnamic acid derivatives occurring in Cichorium endivia vegetables. J Pharm Biomed Anal. 2008 Sep 29; 48 (2):472-6.
58. Pushparaj P, Tan CH, Tan BK. Effects of Averrhoa bilimbi leaf extract on blood glucose and lipids in streptozotocin-diabetic rats. J Ethnopharmacol. 2000 Sep; 72 (1-2):69-76.
59. Süntar I, Küpeli Akkol E, Keles H, Yesilada E, Sarker SD, Baykal T. Comparative evaluation of traditional prescriptions from Cichorium intybus L. for wound healing: stepwise isolation of an active component by in vivo bioassay and its mode of activity. J Ethnopharmacol. 2012 Aug 30; 143 (1):299-309.
60. Pinent M, Blay M, Bladé MC, Salvadó MJ, Arola L, Ardévol A. Grape seed-derived procyanidins have an antihyperglycemic effect in streptozotocin-induced diabetic rats and insulinomimetic activity in insulin-sensitive cell lines. Endocrinology. 2004 Nov; 145 (11):4985-90.
61. Xavier-Filho J, Oliveira A.E.A., Silva L.B. da, Azevedo C.R., Venancio T.M., Machado O.L.T., Oliva M.L., Fernandes K.V.S., Xavier-Neto J. Plant insulin or glucokinin: a conflicting issue. Braz. J. Plant Physiol. 2003 May/Aug; 15 (2):67-78.
62. Andrade-Cetto A, Heinrich M. Mexican plants with hypoglycaemic effect used in the treatment of diabetes. J Ethnopharmacol. 2005 Jul 14; 99 (3):325-48.
63. Diaz-Flores M, Angeles-Mejia S, Baiza-Gutman LA, Medina-Navarro R, Hernández-Saavedra D, Ortega-Camarillo C, Roman-Ramos R, Cruz M, Alarcon-Aguilar FJ. Effect of an aqueous extract of Cucurbita ficifolia Bouché on the glutathione redox cycle in mice with STZ-induced diabetes. J Ethnopharmacol. 2012 Oct 31; 144 (1):101-8.
64. Deutschländer MS, Lall N, Van de Venter M, Hussein AA. Hypoglycemic evaluation of a new triterpene and other compounds isolated from Euclea undulata Thunb. var. myrtina (Ebenaceae) root bark. J Ethnopharmacol. 2011 Feb 16; 133 (3):1091-5.
65. Acosta-Patiño JL, Jiménez-Balderas E, Juárez-Oropeza MA, Díaz-Zagoya JC. Hypoglycemic action of Cucurbita ficifolia on Type 2 diabetic patients with moderately high blood glucose levels. J Ethnopharmacol. 2001 Sep;77(1):99-101.
66. Van de Venter M, Roux S, Bungu LC, Louw J, Crouch NR, Grace OM, Maharaj V, Pillay P, Sewnarian P, Bhagwandin N, Folb P. Antidiabetic screening and scoring of 11 plants traditionally used in South Africa. J Ethnopharmacol. 2008 Sep 2; 119 (1):81-6.
67. Vinson JA, Burnham BR, Nagendran MV. Randomized, double-blind, placebocontrolled, linear dose, crossover study to evaluate the efficacy and safety of a green coffee bean extract in overweight subjects. Diabetes Metab Syndr Obes. 2012;5:21-7.
68. Hamza N, Berke B, Cheze C, Le Garrec R, Lassalle R, Agli AN, Robinson P, Gin H, Moore N. Treatment of high fat diet induced type 2 diabetes in C57BL/6J mice by two medicinal plants used in traditional treatment of diabetes in the east of Algeria. J Ethnopharmacol. 2011 Jan 27; 133 (2):931-3.
69. Ahmed AB, Rao AS, Rao MV. In vitro callus and in vivo leaf extract of Gymnema sylvestre stimulate β-cells regeneration and anti-diabetic activity in Wistar rats. Phytomedicine. 2010 Nov; 17 (13):1033-9.
70. Roman-Ramos R, Flores-Saenz JL, Alarcon-Aguilar FJ. Anti-hyperglycemic effect of some edible plants. J Ethnopharmacol. 1995 Aug 11; 48 (1):25-32.
71. Zhang B, Salituro G, Szalkowski D, Li Z, Zhang Y, Royo I, Vilella D, Díez MT, Pelaez F, Ruby C, Kendall RL, Mao X, Griffin P, Calaycay J, Zierath JR, Heck JV, Smith RG, Moller DE. Discovery of a small molecule insulin mimetic with antidiabetic activity in mice. Science. 1999 May 7;284(5416):974-7.
72. Ghamarian A, Abdollahi M, Su X, Amiri A, Ahadi A, Nowrouzi A. Effect of chicory seed extract on glucose tolerance test (GTT) and metabolic profile in early and late stage diabetic rats. Daru. 2012 Oct 15;20(1):56.
73. DuPont MS, Mondin Z, Williamson G, Price KR. Effect of variety, processing, and storage on the flavonoid glycoside content and composition of lettuce and endive. J Agric Food Chem. 2000 Sep; 48 (9):3957-64.
74. Tousch D, Lajoix AD, Hosy E, Azay-Milhau J, Ferrare K, Jahannault C, Cros G, Petit P. Chicoric acid, a new compound able to enhance insulin release and glucose uptake. Biochem Biophys Res Commun. 2008 Dec 5; 377 (1):131-5.
75. Kamel ZH, Daw I, Marzouk M. Effect of Cichorium endivia leaves on some biochemical parameters in streptozotocin-induced diabetic rats. Aus J Basic and App Sci. 2011; 5 (7):387-96
76. Park KJ, de Oliveira RA, Brod FPR. Drying Operational Parameters Influence on Chicory Roots Drying and Inulin Extraction. Food and Bioproducts Processing. 2007 Sep; 85 (3);184-92
77. Hagel JM, Yeung EC, Facchini PJ. Got milk? The secret life of laticifers. Trends Plant Sci. 2008 Dec;13(12):631-9.
78. Lewinsohn TM. The geographical distribution of plant latex. Chemoecology 1991;2(1):64-8
79. Montagut G, Onnockx S, Vaqué M, Bladé C, Blay M, Fernández-Larrea J, Pujadas G, Salvadó MJ, Arola L, Pirson I, Ardévol A, Pinent M. Oligomers of grape-seed procyanidin extract activate the insulin receptor and key targets of the insulin signaling pathway differently from insulin. J Nutr Biochem. 2010 Jun; 21 (6):476-81.
80. Obanda DN, Hernandez A, Ribnicky D, Yu Y, Zhang XH, Wang ZQ, Cefalu WT. Bioactives of Artemisia dracunculus L. mitigate the role of ceramides in attenuating insulin signaling in rat skeletal muscle cells. Diabetes. 2012 Mar; 61 (3):597-605.
81. Pushparaj PN, Tan BK, Tan CH. The mechanism of hypoglycemic action of the semipurified fractions of Averrhoa bilimbi in streptozotocin-diabetic rats. Life Sci. 2001 Dec 21;70 (5):535-47.
82. Layne J. Characterization and comparison of the chromatographic performance of conventional, polar-embedded, and polar-endcapped reversed-phase liquid chromatography stationary phases. Journal of Chromatography A,2002 957: 149-164.
83. Wolfender JL, Ndjoko K, Hostettmann, K. Liquid chromatography with ultraviolet absorbance-mass spectrometric detection and with nuclear magnetic resonance spectrometry: a powerful combination for the on-line structural investigation of plant metabolites. Journal of Chromatography A 1000 2003: 437-455.
84. Buszewski B, Noga S. Hydrophilic interaction liquid chromatography (HILIC)-a powerful separation technique. Analytical and bioanalytical chemistry, 2012; 402(1): 231-247.
85. Fahey JW, Zalcmann AT, Talalay P. The chemical diversity and distribution of glucosinolates and isothiocyanates among plants. Phytochemistry. 2001 Jan; 56 (1):5-51.

## Claims

1. A botanical extract that provides at least 1 x 10⁴ Insulin Equivalent units in 1 milliliter, wherein the extract is derived from a plant of the plant family Asteraceae, wherein the extract is an aqueous extract, wherein one Insulin Equivalent unit is defined as the minimum amount of extract necessary to increase the growth of insulin receptor substrate 2 (IRS2) overproducing test cells by 1 % of the maximum level of growth of the cells achieved by treatment of the cells with insulin, as measured in an IRS2-specific cell-based assay using 32D Test cells harboring a histidinol-selectable expression vector encoding IRS2 and corresponding 32D Control cells harboring the same histidinol-selectable expression vector lacking the IRS2 coding region, the cells being cultured in IL-3-free medium for 72 hours and 50 nM insulin being used as a positive control normalized to 100%.

2. The extract of claim 1, wherein the extract provides at least 3.6 x 10⁴ Insulin Equivalent units in 1 milliliter.

3. The extract of claim 1, wherein the extract provides from 1 x 10⁴ to 1 x 10⁵ Insulin Equivalent units in 1 milliliter.

4. The extract of any one of claims 1 - 3, which is prepared from Cichorium Endivia var. latifolium.

5. The extract of any one of claims 1 - 3, which is prepared from Lactuca sativa var. crispa or Lactuca sativa var. longifolia.

6. A pharmaceutical composition comprising the extract of any one of claims 1 - 5.

7. A nutritional supplement comprising the extract of any one of claims 1 - 5.

8. The pharmaceutical composition of claim 6 for use in treating an insulin receptor substrate (IRS) mediated disease or condition, wherein the IRS mediated disease or condition is diabetes, pre-diabetes, metabolic syndrome, insulin resistance, or dementia.

9. The nutritional supplement of claim 7 for use in treating an IRS mediated disease or condition, wherein the IRS mediated disease or condition is diabetes, pre-diabetes, metabolic syndrome, insulin resistance, or dementia.

10. The pharmaceutical composition for use of claim 8 or the nutritional supplement for use of claim 9, which use further comprises an antidiabetic agent, insulin, metformin, exenatide, vildagliptin, sitagliptin, a DPP4 inhibitor, meglitinide, exendin-4, liraglutide, or a GLP1 agonist.

## Patentansprüche

1. Pflanzenextrakt, der mindestens 1 x 10⁴ Insulin-Äquivalent-Einheiten in 1 Milliliter bereitstellt, wobei der Extrakt aus einer Pflanze der Pflanzenfamilie Asteraceae stammt, wobei der Extrakt ein wässriger Extrakt ist, wobei eine Insulin-Äquivalent-Einheit als die Mindestmenge an Extrakt definiert ist, die erforderlich ist, um das Wachstum von Testzellen, die Insulinrezeptorsubstrat-2 (IRS2) überproduzieren, um 1% des maximalen Wachstumsniveaus der Zellen zu erhöhen, das durch Behandlung der Zellen mit Insulin erreicht wird, gemessen in einem IRS2-spezifischen zellbasierten Assay unter Verwendung von 32D-Testzellen, die einen Histidinol-selektierbaren Expressionsvektor enthalten, der IRS2 codiert, und entsprechenden 32D-Kontrollzellen, die denselben Histidinol-selektierbaren Expressionsvektor ohne die IRS2-codierende Region enthalten, wobei die Zellen 72 Stunden lang in IL-3-freiem Medium kultiviert werden und 50 nM Insulin als auf 100 % normierte Positivkontrolle verwendet werden.

2. Extrakt nach Anspruch 1, wobei der Extrakt mindestens 3,6 x 10⁴ Insulin-Äquivalent-Einheiten in 1 Milliliter bereitstellt.

3. Extrakt nach Anspruch 1, wobei der Extrakt 1 x 10⁴ bis 1 x 10⁵ Insulin-Äquivalent-Einheiten in 1 Milliliter bereitstellt.

4. Extrakt nach einem der Ansprüche 1 - 3, der aus Cichorium Endivia var. latifolium hergestellt wird.

5. Extrakt nach einem der Ansprüche 1 - 3, der aus Lactuca sativa var. crispa oder Lactuca sativa var. longifolia hergestellt wird.

6. Pharmazeutische Zusammensetzung, umfassend den Extrakt nach einem der Ansprüche 1 - 5.

7. Nahrungsergänzungsmittel, umfassend den Extrakt nach einem der Ansprüche 1 - 5.

8. Pharmazeutische Zusammensetzung nach Anspruch 6 zur Verwendung bei der Behandlung einer durch Insulinrezeptorsubstrat (IRS) vermittelten Erkrankung oder eines durch Insulinrezeptorsubstrat (IRS) vermittelten Zustands, wobei die IRS-vermittelte Erkrankung oder der IRS-vermittelte Zustand Diabetes, Prädiabetes, metabolisches Syndrom, Insulinresistenz oder Demenz ist.

9. Nahrungsergänzungsmittel nach Anspruch 7 zur Verwendung bei der Behandlung einer IRS-vermittelten Erkrankung oder eines IRS-vermittelten Zustands, wobei die IRS-vermittelte Erkrankung oder der IRS-vermittelte Zustand Diabetes, Prädiabetes, metabolisches Syndrom, Insulinresistenz oder Demenz ist.

10. Pharmazeutische Zusammensetzung zur Verwendung nach Anspruch 8 oder Nahrungsergänzungsmittel zur Verwendung nach Anspruch 9, wobei die Verwendung ferner ein Antidiabetikum, Insulin, Metformin, Exenatid, Vildagliptin, Sitagliptin, einen DPP4-Inhibitor, Meglitinid, Exendin-4, Liraglutid oder einen GLP1-Agonisten umfasst.

## Revendications

1. Un extrait botanique qui fournit au moins 1 x 10⁴ unités équivalentes insuline dans 1 millilitre, dans lequel extrait est dérivé d'une plante de la famille de plantes Asteraceae, dans lequel extrait est un extrait aqueux, dans lequel une unité équivalente insuline est définie comme la quantité minimale d'extrait nécessaire pour augmenter la croissance de cellules test surproduisant le substrat du récepteur de l'insuline 2 (IRS2) de 1 % du niveau maximal de croissance des cellules obtenu par traitement des cellules avec de l'insuline, tel que mesuré dans un essai cellulaire spécifique IRS2 utilisant des cellules test 32D contenant un vecteur d'expression sélectionnable à l'histidinol codant IRS2 et des cellules témoins 32D correspondantes contenant le même vecteur d'expression sélectionnable à l'histidinol dépourvu de la région codante IRS2, les cellules étant cultivées dans un milieu sans IL-3 pendant 72 heures et 50 nM d'insuline étant utilisé comme témoin positif normalisé à 100 %.

2. L'extrait selon la revendication 1, dans lequel extrait fournit au moins 3,6 x 10⁴ unités équivalentes insuline dans 1 millilitre.

3. L'extrait selon la revendication 1, dans lequel extrait fournit de 1 x 10⁴ à 1 x 10⁵ unités équivalentes insuline dans 1 millilitre.

4. L'extrait selon l'une quelconque des revendications 1 à 3, qui est préparé à partir de Cichorium Endivia var. latifolium.

5. L'extrait selon l'une quelconque des revendications 1 à 3, qui est préparé à partir de Lactuca sativa var. crispa ou Lactuca sativa var. longifolia.

6. Une composition pharmaceutique comprenant l'extrait selon l'une quelconque des revendications 1 à 5.

7. Un complément nutritionnel comprenant l'extrait selon l'une quelconque des revendications 1 à 5.

8. La composition pharmaceutique selon la revendication 6 pour une utilisation dans le traitement d'une maladie ou affection médiée par le substrat du récepteur de l'insuline (IRS), laquelle maladie ou affection médiée par IRS est le diabète, le prédiabète, le syndrome métabolique, la résistance à l'insuline ou la démence.

9. Le complément nutritionnel selon la revendication 7 pour une utilisation dans le traitement d'une maladie ou affection médiée par IRS, laquelle maladie ou affection médiée par IRS est le diabète, le prédiabète, le syndrome métabolique, la résistance à l'insuline ou la démence.

10. La composition pharmaceutique pour utilisation selon la revendication 8 ou le complément nutritionnel pour utilisation selon la revendication 9, laquelle utilisation comprend en outre un agent antidiabétique, de l'insuline, de la metformine, de l'exénatide, de la vildagliptine, de la sitagliptine, un inhibiteur de DPP4, un meglitinide, de l'exendin-4, du liraglutide ou un agoniste du GLP1.
